# EUROPEAN PATENT SPECIFICATION

(11) **EP 4 087 945 B1**
(45) Date of publication and mention of the grant of the patent: **06.03.2024**
(21) Application number: 21703116.0
(22) Date of filing: 08.01.2021
(51) Int. Cl.: C12Q 1/6874

(54) **METHODS FOR DETERMINING A LOCATION OF A TARGET NUCLEIC ACID IN A BIOLOGICAL SAMPLE**
VERFAHREN ZUM BESTIMMEN DES STANDORTES EINER ZIEL-NUKLEINSÄURE IN EINER BIOLOGISCHEN PROBE
PROCÉDÉS DE DÉTERMINATION D'UN EMPLACEMENT D'UN ACIDE NUCLÉIQUE CIBLE DANS UN ÉCHANTILLON BIOLOGIQUE

(30) Priority: 10.01.2020 US 202062959765 P; 04.03.2020 US 202062985292 P; 16.09.2020 US 202063079189 P; 05.10.2020 US 202063087553 P
(43) Date of publication of application: 16.11.2022
(60) Divisional application: 24150449.7
(73) Proprietor: 10X Genomics, Inc., Pleasanton, CA 94588-3260 (US)
(72) Inventor: STOECKIUS, Marlon, 11852 Stockholm (SE); CHELL, James Michael, 12064 Stockholm (SE); UYTINGCO, Cedric, Pleasanton, California 94588-3260 (US); BAVA, Felice Alessio, Pleasanton, California 94588-3260 (US); CHEW, Jennifer, Pleasanton, California 94588-3260 (US)
(74) Representative: D Young & Co LLP
(86) International application number: PCT/US2021/012659
(87) International publication number: WO 2021/142233

(56) References cited:
- WO-A1-2016/168825
- ASP MICHAELA ET AL: "Spatial detection of fetal marker genes expressed at low level in adult human heart tissue", SCIENTIFIC REPORTS, vol. 7, no. 1, 11 October 2017 (2017-10-11), XP055795937, DOI: 10.1038/s41598-017-13462-5 Retrieved from the Internet: URL:http://www.nature.com/articles/s41598- 017-13462-5>
- VICKOVIC SANJA ET AL: "Massive and parallel expression profiling using microarrayed single-cell sequencing", NATURE COMMUNICATIONS, vol. 7, no. 1, 14 October 2016 (2016-10-14), pages 1-9, XP055795938, DOI: 10.1038/ncomms13182 Retrieved from the Internet: URL:http://www.nature.com/articles/ncomms1 3182>
- STÅHL PATRIK L ET AL: "Visualization and analysis of gene expression in tissue sections by spatial transcriptomics", SCIENCE, AMERICAN ASSOCIATION FOR THE ADVANCEMENT OF SCIENCE, US, vol. 353, no. 6294, 1 July 2016 (2016-07-01), pages 78-82, XP002784680, ISSN: 1095-9203, DOI: 10.1126/SCIENCE.AAF2403 -& PATRIK L. STÅHL ET AL: "Supplementary Materials for Visualization and analysis of gene expression in tissue sections by spatial transcriptomics", SCIENCE, vol. 353, no. 6294, 30 June 2016 (2016-06-30), pages 1-40, XP055653296, US ISSN: 0036-8075, DOI: 10.1126/science.aaf2403 Retrieved from the Internet: URL:www.sciencemag.org/content/353/6294/78 /suppl/DC1>

## Description

### BACKGROUND

Cells within a tissue have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling, and cross-talk with other cells in the tissue.

Spatial heterogeneity has been previously studied using techniques that typically provide data for a handful of analytes in the context of intact tissue or a portion of a tissue (e.g., tissue section), or provide significant analyte data from individual, single cells, but fails to provide information regarding the position of the single cells from the originating biological sample (e.g., tissue).

Fixed-formalin paraffin embedded (FFPE) samples provide a useful way to archive biological samples obtained from patients. Analytes can be successfully retrieved and analyzed from FFPE samples after a substantial amount of storage time (e.g., years) without any significant effect on the extracted analytes.

Asp et al (Scientific Reports Vol 7 No. 1, DOl: 10.1 038/s41598-017-13462-5, 2017) disclose that spatial detection of fetal marker genes with low expression levels in adult human heart tissue could potentially differentiate disease states in order to improve therapy.

Vickovic et al (Nature Communications, Vol 7 No.1, pages 1-9, 2016) disclose a method enabling massive microarray-based barcoding of expression patterns in single cells (MASC-seq).

Stahl et al (Science, Vol. 353, No. 6294, pages 78-82, 2016) disclose the method spatial transcriptomics that allows visualization and quantitative analysis of the transcriptome with spatial resolution in individual tissue sections.

WO2016/168825 discloses methods and compositions for profiling the spatial distribution of a biological molecules in a sample.

### SUMMARY

The preservation of biological samples is useful since preservation maintains cellular morphology and details of biological samples. Preserving biological samples also creates an archived library that can be further examined or interrogated retrospectively. Different types of preservation methods are known, including, for example formalin-fixed paraffin-embedded (FFPE) fixation, paraformaldehyde fixation (PFA), and acetone fixation.

At present methods, compositions, and kits for spatially capturing analytes from a biological sample (e.g., a fresh-frozen) biological sample have been previously described. However, there remains a need to spatially capture analytes from preserved biological samples, such as fixed biological samples. The present disclosure features methods, compositions, and kits for the spatial capture of analytes on an array comprising a plurality of capture probes, where a capture probe of the plurality of capture probes includes a capture domain and a spatial barcode.

The present invention provides a method for de-crosslinking a fixed biological sample for determining the location of a target nucleic acid in the fixed biological sample, the method comprising:
(a) providing a spatial array comprising a plurality of capture probes, wherein a capture probe of the plurality of capture probes comprises: (i) a spatial barcode and (ii) a capture domain;
(b) de-crosslinking one or more crosslinks in the fixed biological sample, wherein the de-crosslinking comprises heating the fixed biological sample such that the capture domain hybridizes to a proxy of the target nucleic acid;
(c) contacting the fixed biological sample with:
   (i) a first probe, wherein the first probe comprises in a 5' to 3' direction: a first functional domain and a first sequence that is substantially complementary to a portion of the target nucleic acid, and
   (ii) a second probe, wherein the second probe comprises in a 5' to 3' direction: a 5' phosphate, a second sequence that is substantially complementary to a portion of the target nucleic acid and a capture sequence that is substantially complementary to the capture domain of the capture probe;
(d) hybridizing the first probe and the second probe to the target nucleic acid and ligating the 3' end of the first probe to the 5' end of the second probe to generate the proxy of the target nucleic acid;
(e) hybridizing the proxy of the target nucleic acid to the capture domain of the capture probe, extending the capture probe using the proxy as a template thereby generating an extended capture probe, and generating a second strand hybridized to the extended capture probe, wherein the second strand comprises a sequence complementary to the spatial barcode and the proxy of the target nucleic acid; and
(f) determining (i) the sequence of the spatial barcode, or a complement thereof, and (ii) all or a portion of the proxy of the target nucleic acid, or a complement thereof, and using the determined sequences of (i) and (ii) to identify the location of the target nucleic acid in the fixed biological sample.

Disclosed though not part of the invention are methods for determining a location of a target nucleic acid in a fixed biological sample, the method including: (a) a fixed biological sample on a spatial array including a plurality of capture probes, wherein a capture probe of the plurality of capture probes includes (i) a spatial barcode and (ii) a capture domain; (b) de-crosslinking one or more crosslinks in the fixed biological sample, such that a capture domain binds to the target nucleic acid or a proxy thereof; (c) extending the capture probe to generate a nucleic acid sequence that is complementary to a portion of the target nucleic acid; (d) generating a second strand hybridized to the extended capture probe, wherein the second strand includes a sequence complementary to the spatial barcode and the nucleic acid sequence corresponding to a portion of the sequence in the target nucleic acid; and (e) determining (i) all or a part of the sequence of the spatial barcode, or a complement thereof, and (ii) all or a part of the portion of the sequence of the target nucleic acid, or a complement thereof, and using the determined sequences of (i) and (ii) to identify the location of the target nucleic acid in the fixed biological sample.

In some embodiments, the de-crosslinking step includes heating the fixed biological sample. In some embodiments, the de-crosslinking step includes the performance of a chemical reaction or the use of an enzyme.

In some embodiments, the fixed biological sample is a formalin-fixed paraffin-embedded (FFPE) biological sample.

In some embodiments, the de-crosslinking step includes the use of Tris-EDTA (TE) buffer. In some embodiments, the TE buffer has a pH of about 7.5 to about 8.5. In some embodiments, the TE buffer has a temperature of about 65 °C to about 75 °C, and is contacted with the FFPE biological sample for about 10 minutes to about 200 minutes.

In some embodiments, the fixed biological sample is a paraformaldehyde fixed biological sample. In some embodiments, the de-crosslinking step includes the use of Tris-HCl buffer with a pH of about 8.5 to 9.5 at a temperature of about 55°C to about 65°C, and is contacted with the paraformaldehyde fixed biological sample for about 10 minutes to about 200 minutes.

In some embodiments, includes, after the de-crosslinking step, a step of permeabilizing the fixed biological sample. In some embodiments, the step of permeabilizing the fixed biological sample includes the use of a protease, wherein the protease is pepsin or proteinase K.

In some embodiments, the plurality of capture probes includes an additional capture probe including, in a 5' to a 3' direction: a spatial barcode and a poly(T) capture domain.

In some embodiments, the target nucleic acid includes RNA. In some embodiments, the RNA is mRNA.

In some embodiments, the method includes, between steps (b) and (c), a step of incubating the fixed biological sample with a 5' to 3' single-stranded DNA exonuclease. In some embodiments, the method includes, between steps (b) and (c), a step of incubating the fixed biological sample with a 5' to 3' single-stranded DNA exonuclease and a double-stranded DNA endonuclease, wherein the single-stranded DNA exonuclease is T7 endonuclease or RecJε. In some embodiments, the method includes, incubating the fixed biological sample with a restriction endonuclease having an AT-rich restriction endonuclease recognition sequence.

In some embodiments, step (d) includes, the steps of: adding a homonucleotide sequence to the 3' end of the capture probe; and ligating a sequencing or PCR handle to a 3' end of the homonucleotide sequence using splint ligation or a DNA polymerase extension reaction. In some embodiments, the step of adding the homonucleotide sequence is performed using terminal deoxynucleotidyl transferase (TdT).

In some embodiments, the splint ligation includes the use of a partially-double stranded nucleic acid including a first oligonucleotide hybridized to a second oligonucleotide, wherein: the first oligonucleotide includes a functional domain; the second oligonucleotide includes in a 5' to a 3' direction: a poly(G) sequence and a sequence that is substantially complementary to the functional domain; the poly(G) sequence of the second oligonucleotide binds to the poly(C) sequence of the capture probe; and the partially-double stranded nucleic acid has a 5' overhang on the second strand, wherein the 5' overhang includes at least a portion of the poly(G) sequence. In some embodiments, the functional domain is a sequencing or PCR handle and the splint ligation results in the addition of the sequencing or PCR handle to the 3' end of the capture domain. In some embodiments, step (d) includes: hybridizing a primer including a sequence substantially complementary to the sequencing or PCR handle in the capture probe; and extending the primer to generate the second strand.

In some embodiments, step (d) includes, at least in part, the step of attaching a 5' adenylated adaptor to the 3' end of the capture probe using a ligase. In some embodiments, the ligase is 5' App DNA/RNA ligase, T4 RNA ligase 2, or any other single-stranded DNA or RNA ligase. In some embodiments, the 5' adenylated adaptor includes a sequencing handle. In some embodiments, step (d) includes: hybridizing a primer including a sequence substantially complementary to the sequencing or PCR handle in the capture probe; and extending the primer to generate the second strand.

In some embodiments, wherein step (d) includes, at least in part, the steps of: adding a poly(A) sequence to the 3' end of the capture probe; and ligating an adaptor to a 3' end of the poly(A) sequence in the capture probe. In some embodiments, the poly(A) sequence is a ribonucleic acid sequence and the ligating is performed using an RNA ligation.

In some embodiments, the adaptor includes a sequencing or PCR handle. In some embodiments, step (d) includes: hybridizing a primer including a sequence substantially complementary to the sequencing or PCR handle in the capture probe; and extending the primer to generate the second strand. In some embodiments, the step of extending the primer to generate the second strand is performed using a DNA polymerase. In some embodiments, the step of extending the primer to generate the second strand is performed using a DNA polymerase that can use RNA bases as a substrate.

In some embodiments, step (d) includes hybridizing one or more primers, wherein a primer of the one or more primers includes a random sequence and a functional domain, wherein the random sequence hybridizes to a sequence in the extended capture probe that is substantially complementary to the random sequence. In some embodiments, the functional domain is a sequencing handle. In some embodiments, step (d) includes extending the primer to generate the second strand.

In some embodiments, step (d) includes, at least in part, a step of ligating an adaptor to the 3' end of the capture probe. In some embodiments, the adaptor includes a sequencing handle. In some embodiments, step (d) includes: hybridizing a primer including a sequence substantially complementary to the sequencing handle in the capture probe; and extending the primer to generate the second strand.

In some embodiments, the functional domain includes a template switching oligonucleotide sequence followed by a random sequence. In some embodiments, the functional domain followed by a random sequence including SEQ ID NO: 4 or SEQ ID NO: 5. In some embodiments, the method includes a template switching oligonucleotide.

In some embodiments, the method includes imaging the fixed biological sample. In some embodiments, the fixed biological sample is an FFPE tissue section or a paraformaldehyde fixed tissue section. In some embodiments, the capture probe includes a unique molecular identifier (UMI) positioned 5' to the capture domain in the capture probe.

In some embodiments, the method includes after step (b) contacting the fixed biological sample with a first probe, wherein the first probe includes in a 5' to a 3' direction: a first functional domain and a first sequence that is substantially complementary to a portion of the target nucleic acid.

In some embodiments, the method includes (i) contacting the fixed biological sample with a second probe, wherein the second probe includes in a 5' to a 3' direction: a second sequence that is substantially complementary to a portion of the target nucleic acid and an analyte capture sequence, wherein the capture domain binds to the analyte capture sequence of the proxy of the target nucleic acid; a 5' end of the second probe includes a 5' phosphate; and the first sequence and the second sequence bind to the target nucleic acid; (ii) ligating the 3' end of the first probe to the 5' end of the second probe to generate a proxy of the target nucleic acid; (iii) extending a 3' end of the capture probe to generate a sequence that corresponds to a portion of the proxy of the target nucleic acid; (iv) generating a second strand hybridized to the extended capture probe, wherein the second strand includes a sequence complementary to the spatial barcode and a sequence complementary to a portion of the proxy of the target nucleic acid; and (v) determining (i) all or a part of the sequence of the spatial barcode, or a complement thereof, and (ii) all or a part of the portion of the proxy of the target nucleic acid, or a complement thereof, and using the determined sequences of (i) and (ii) to identify the location of the target nucleic acid in the fixed biological sample.

In some embodiments, the first functional domain is a first sequencing handle and wherein the analyte capture sequence is substantially complementary to the capture domain, or a portion thereof.

In some embodiments, the ligating of the 3' end of the first probe to a 5' end of the second probe is performed using a ligase.

In some embodiments, the method includes, between steps (b) and (c), a step of treating the FFPE biological sample with an RNAse.

In some embodiments, step (e) includes: hybridizing a primer including at least a portion of the first functional domain to the capture probe; and extending the primer to generate the second strand. In some embodiments, the 3' end of the first probe includes a 3' diribo sequence.

In some embodiments, the method includes after step (a) adding an analyte capture sequence to the target nucleic acid. In some embodiments, adding the analyte capture sequence is performed using terminal deoxynucleotidyl transferase (TdT) or poly(A) polymerase.

Disclosed though not part of the invention are method for determining a location of a target nucleic acid in a fixed biological sample, including (a) contacting the fixed biological sample with a first and a second probe, where the first probe includes in a 5' to a 3' direction: a first functional domain and a first sequence that is substantially complementary to a portion of the target nucleic acid; the second probe includes in a 5' to a 3' direction: a second sequence that is substantially complementary to a portion of the target nucleic acid and an analyte capture sequence; a 5' end of the second probe comprises a 5' phosphate; and the first sequence and the second sequence bind to the target nucleic acid; (b) ligating the 3' end of the first probe to the 5' end of the second probe to generate a proxy of the target nucleic acid; (c) contacting the fixed biological sample to an array including a capture probe including (i) a spatial barcode and (ii) a capture domain including a sequence substantially complementary to the analyte capture sequence, where the capture domain binds to the analyte capture sequence of the proxy of the target nucleic acid; (d) extending a 3' end of the capture probe to generate a sequence that corresponds to a portion of the proxy of the target nucleic acid; (e) generating a second strand hybridized to the extended capture probe, wherein the second strand includes a sequence complementary to the spatial barcode and a sequence complementary to a portion of the proxy of the target nucleic acid; and (f) determining (i) all or a part of the sequence of the spatial barcode, or a complement thereof, and (ii) all or a part of the portion of the proxy of the target nucleic acid, or a complement thereof, and using the determined sequences of (i) and (ii) to identify the location of the target nucleic acid in the fixed biological sample.

Disclosed though not part of the invention are methods for determining a location of a target nucleic acid in a formalin-fixed paraffin-embedded (FFPE) biological sample, including (a) a FFPE biological sample on a spatial array including a plurality of capture probes, wherein a capture probe of the plurality of capture probes includes (i) a spatial barcode and (ii) a capture domain; (b) de-crosslinking one or more formaldehyde crosslinks in the FFPE biological sample, such that the capture domain binds to the target nucleic acid; (c) extending the capture probe to generate a nucleic acid sequence that is complementary to a portion of the target nucleic acid; (d) contacting the extended capture probe with a nucleic acid substantially complementary to the extended capture probe with a labeled nucleic acid such that the labeled nucleic acid binds to the extended capture probe, or portion thereof; and (e) detecting the labeled nucleic acid to identify the location of the target nucleic acid in the FFPE biological sample.

Disclosed though not part of the invention are methods for determining a location of a target nucleic acid in a paraformaldehyde fixed biological sample, including (a) a paraformaldehyde fixed biological sample on a spatial array comprising a plurality of capture probes, wherein a capture probe of the plurality of capture probes includes (i) a spatial barcode and (ii) a capture domain; (b) decrosslinking paraformaldehyde crosslinks in the PFA biological sample, such that the capture domain binds to the target nucleic acid; (c) extending the capture probe to generate a nucleic acid sequence that is complementary to a portion of the target nucleic acid; (d) generating a second strand hybridized to the extended capture probe, wherein the second strand comprises a sequence complementary to the spatial barcode and the nucleic acid sequence corresponding to a portion of the sequence in the target nucleic acid; and (d) determining (i) all or a part of the sequence of the spatial barcode, or a complement thereof, and (ii) all or a part of the portion of the sequence of the target nucleic acid, or a complement thereof, and using the determined sequences of (i) and (ii) to identify the location of the target nucleic acid in the paraformaldehyde fixed biological sample.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

### DESCRIPTION OF DRAWINGS

The following drawings illustrate certain embodiments of the features and advantages of this disclosure. Like reference symbols in the drawings indicate like elements.

The following drawings illustrate certain embodiments of the features and advantages of this disclosure. Like reference symbols in the drawings indicate like elements.
**FIG. 1** is a schematic diagram showing an example of a barcoded capture probe, as described herein.
**FIGs. 2A-C** show spatial clustering using different mouse brain samples. Data obtained from a fresh frozen biological sample is shown in **FIG. 2A****,** data obtained from an FFPE biological sample without decrosslinking is shown in **FIG. 2B****,** and data obtained from an FFPE biological sample with decrosslinking is shown in **FIG. 2C****.**
**FIGs. 3A-C** show spatial clustering of an individual gene (PRKCD) using different mouse brain samples. Data obtained from a fresh frozen biological sample is shown in **FIG. 3A****,** data obtained from an FFPE biological sample without decrosslinking is shown in **FIG. 3B****,** and data obtained from an FFPE biological sample with decrosslinking is shown in **FIG. 3C****.**
**FIGs. 4A-C** show sample correlation plots of data obtained from different biological samples. **FIG. 4A** is a correlation plot of data obtained from a fresh frozen sample and data obtained from a fresh frozen sample. **FIG. 4B** is a correlation plot of data obtained from a decrosslinked FFPE biological sample and data obtained from a decrosslinked FFPE biological sample. **FIG. 4C** is a correlation plot of data obtained from a decrosslinked FFPE biological sample and data obtained from a fresh frozen biological sample.
**FIGs. 5A-B** show successful spatial clustering from a decrosslinked 7-year old metaplastic carcinoma FFPE biological sample. **FIG. 5A** is a tissue plot with spots colored by UMI count. **FIG. 5B** is a t-SNE projection of spots colored by UMI counts.
**FIGs. 6A-B** show representations of successful spatial clustering from a decrosslinked 7-year old metaplastic carcinoma FFPE biological sample. **FIG. 6A** is a representation of a tissue plot with spots colored by automated clustering. **FIG. 6B** is a representation of a t-SNE projection of spots colored by automated clustering.
**FIG. 7** is a schematic diagram showing addition of a poly(C) sequence with a terminal transferase to the 3' end of an extended capture probe, followed by ligation of a sequencing handle via a splint oligonucleotide.
**FIGs. 8A-B** are replicate experiments showing cDNA generated from fresh frozen and decrosslinked FFPE biological samples and prepared using the method depicted in **FIG. 7****.**
**FIG. 9** shows successful spatial analysis with decrosslinked FFPE biological samples prepared using the method depicted in **FIG. 7****.**
**FIG. 10** shows successful spatial clustering with decrosslinked FFPE biological samples prepared using the method depicted in **FIG. 7****.**
**FIG. 11** is a schematic diagram showing a method that can be used to generate a second strand after reverse transcription with random primers including a sequencing handle.
**FIG. 12** shows a sequencing library prepared from a decrosslinked FFPE biological sample prepared using the method depicted in **FIG. 10****.**
**FIG. 13** shows successful spatial analysis performed on a decrosslinked FFPE biological sample prepared using the method depicted in **FIG. 11****.**
**FIG. 14** shows successful spatial clustering performed on a decrosslinked FFPE biological sample prepared using the method depicted in **FIG. 11****.**
**FIG. 15** is a schematic diagram showing the ligation a 5' adenylated adaptor including a sequencing handle to the 3' end of an extended capture probe after reverse transcription.
**FIG. 16** is a schematic diagram showing the tailing of the 3' end of an extended capture probe with a poly(A) ribonucleic acid sequence and ligating a sequencing adaptor with an RNA ligase.
FIGs. 17A-B are fluorescently-labeled cDNA images of captured nucleic acids in 1% paraformaldehyde (PFA) fixed biological samples with either no decrosslinking (**FIG. 17A**) or decrosslinked 1% PFA fixed biological samples (**FIG. 17B**) under various permeabilization durations.
**FIG. 18** is a graph showing signal to noise (SNR) of cDNA fluorescence to background fluorescence in decrosslinked PFA fixed biological samples.
FIGs. 19A-B are fluorescently-labeled cDNA images of captured nucleic acids in 2% PFA fixed biological samples with either no decrosslinking (**FIG. 19A**) or 2% PFA fixed biological samples with decrosslinking (**FIG. 19B**) under various permeabilization durations.
**FIGs. 20A-B** are fluorescently-labeled cDNA images of captured nucleic acids in 4% PFA fixed biological samples with either no decrosslinking (**FIG. 20A**) or 4% PFA fixed biological samples with decrosslinking (**FIG. 20B**) under various permeabilization durations.
FIGs. 21A-B are graphs showing SNR of cDNA fluorescence to background fluorescence in decrosslinked 1%, 2%, and 4% PFA fixed biological samples (**FIG. 21A**) and decrosslinked 4% PFA fixed biological samples alone (**FIG. 21B**).
**FIGs. 22A-B** are fluorescently-labeled cDNA images of captured nucleic acids in 4% PFA fixed cardiac perfused biological samples with either no decrosslinking (**FIG. 22A**) or decrosslinked 4% PFA fixed cardiac perfused biological samples (**FIG. 22B**) under various permeabilization durations.
**FIGs. 23A-B** are graphs showing cDNA fluorescence in decrosslinked 4% PFA fixed cardiac perfused biological samples (**FIG. 23A**) and background cDNA fluorescence in decrosslinked 4% PFA fixed cardiac perfused biological samples (**FIG. 23B**)**.**
**FIG. 24** is a graph showing SNR of cDNA fluorescence to background fluorescence in decrosslinked 4% PFA fixed cardiac perfused biological samples.
**FIGs. 25A-D** are graphs showing sequencing sensitivity metrics (Fraction of reads usable (**FIG. 25A**); Reads mapped confidently to the transcriptome (**FIG. 25B**); Median genes per spot (**FIG. 25C**); and Median UMI counts per spot (**FIG. 25D**) in decrosslinked 1%, 2%, and 4% PFA fixed biological samples, decrosslinked 4% PFA fixed cardiac perfused biological samples, and control samples
**FIGs. 26A-C** shows spatial clustering analysis performed on a methanol control biological sample (**FIG. 26A**), a non-decrosslinked 4% PFA fixed biological sample (**FIG. 26B**), and a decrosslinked 4% PFA fixed biological sample (**FIG. 26C**).
**FIGs. 27A-C** shows spatial clustering analysis performed on a methanol control perfused biological sample (**FIG. 27A**), a non-decrosslinked 4% PFA fixed perfused biological sample (**FIG. 27B**), and a decrosslinked 4% PFA fixed perfused biological sample (**FIG. 27C**).
**FIG. 28** shows sequencing sensitivity metrics under various conditions.
**FIG. 29** shows sequencing library quality metrics under various conditions.

### DETAILED DESCRIPTION

The preservation of biological samples is useful since preservation maintains cellular morphology and details of biological samples. Preserving biological samples also creates an archived library that can be further examined or interrogated retrospectively. Different types of preservation methods are known, including, for example formalin-fixed paraffin-embedded (FFPE) fixation and paraformaldehyde fixation (PFA).

Fixed (e.g., FFPE, PFA) processed biological samples are advantageous because they can be stored long term (e.g., years) at ambient (e.g., room temperature), thus reducing costs associated with storing biological samples (e.g., frozen tissue) at ultra-low temperatures. Further, fixed (e.g., FFPE, PFA) biological samples can be stored long-term (e.g., years) with no significant effect on the quantity and/or quality of extracted analytes (e.g., nucleic acid, protein, etc.) (See, Kokkat, T.J., et. al., Archived Formalin-Fixed Paraffin-Embedded (FFPE) Blocks: A Valuable Underexploited Resource for Extraction of DNA, RNA, and Protein, Biopreserv Biobank, 11(2), 101-106, 10.1089/bio.2012.0052 (2013)).

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction).

Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a ligation product. The ligation product can function as a proxy for the target nucleic acid. For example, the proxy of the target nucleic acid (e.g., ligation product) can include an analyte capture sequence that can bind (e.g., hybridize) to the capture domain of a capture probe. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product (e.g., proxy of the target nucleic acid) is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed..." of WO 2020/176788 and/or U.S. Patent Application Publication No. 2020/0277663. See also, e.g., the Visium Spatial Gene Expression Reagent Kits User Guide (e.g., Rev C, dated June 2020), and/or the Visium Spatial Tissue Optimization Reagent Kits User Guide (e.g., Rev C, dated July 2020).

In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in PCT Application No. 2020/061064.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2020/061108.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of WO 2020/123320, PCT Application No. 2020/061066. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### Spatial Analysis in Fixed Biological Samples

The preservation of biological samples is useful since preservation maintains cellular morphology and details of biological samples. Preserving biological samples also creates an archived library that can be further examined or interrogated retrospectively. Different types of preservation methods are known, including, for example formalin-fixed paraffin-embedded (FFPE) fixation and paraformaldehyde fixation (PFA).

Fixed (e.g., FFPE, PFA) processed biological samples are advantageous because they can be stored long term (e.g., years) at ambient (e.g., room temperature), thus reducing costs associated with storing biological samples (e.g., frozen tissue) at ultra-low temperatures. Further, fixed (e.g., FFPE, PFA) biological samples can be stored long-term (e.g., years) with no significant effect on the quantity and/or quality of extracted analytes (e.g., nucleic acid, protein, etc.) (See, Kokkat, T.J., et. al., Archived Formalin-Fixed Paraffin-Embedded (FFPE) Blocks: A Valuable Underexploited Resource for Extraction of DNA, RNA, and Protein, Biopreserv Biobank, 11(2), 101-106, 10.1089/bio.2012.0052 (2013)).

Provided herein are methods for determining the location of analytes present in a fixed (e.g., FFPE, PFA) biological sample. While accessing analytes (e.g., RNA) in fixed (e.g., FFPE, PFA) biological samples can be performed, efficiently capturing and/or amplifying analytes from fixed (e.g., FFPE, PFA) biological samples for spatial analysis can present challenges. For example, RNA (e.g., mRNA) in fixed (e.g., FFPE, PFA) biological samples is generally inaccessible for spatial analysis due to crosslinks (e.g., formaldehyde-induced crosslinks) to other analytes (e.g., nucleic acid, protein, etc.). In another example, analytes (e.g., mRNA) can be altered such that the mRNA does not have a poly(A) tail or may be missing a poly(A) tail. Such alterations can arise from transcript fragmentation, degradation, and/or formaldehyde modifications during the preservation process (Evers, D.L., et. al., The effect of formaldehyde fixation on RNA: optimization of formaldehyde adduct removal, J. MolDiagn. 13(3): 282-8, doi: 10.1016/j.jmoldx.2011.01.010 (2011)).

As such, capture and/or amplification efficiency of such analytes (e.g., mRNA) with spatial arrays described herein can be reduced for any of the above reasons. Provided herein are methods for improving the efficiency of capture and/or amplification of analytes (e.g., nucleic acid, any of the nucleic acid analytes described herein) in FFPE biological samples.

Provided herein are methods for determining a location of a target nucleic acid in a fixed (e.g., FFPE, paraformaldehyde, acetone, methanol) biological sample that include: contacting the FFPE biological sample with an array including a plurality of capture probes, where a capture probe of the plurality of capture probes includes a spatial barcode and a capture domain ; de-crosslinking one or more crosslinks in the fixed (e.g., FFPE, paraformaldehyde)biological sample, such that the capture domain specifically binds to the target nucleic acid; extending the capture probe to add a sequence that is complementary to a portion of the target nucleic acid; generating a second strand hybridized to the extended capture probe, where the second strand includes a sequence complementary to the spatial barcode and the nucleic acid sequence corresponding to a portion of the sequence in the target nucleic acid; and determining all or a part of the sequence of the spatial barcode, or a complement thereof, and all or a part of the portion of the sequence of the target nucleic acid, or a complement thereof, and using the determined sequences of all or a part of the sequence of the spatial barcode, or complement thereof, and all or a part of the portion of the sequence of the target nucleic acid, or complement thereof, to identify the location of the target nucleic acid in the fixed (e.g., FFPE, paraformaldehyde) biological sample.

Also provided herein are methods for determining a location of a target nucleic acid in a paraformaldehyde (PFA) biological sample including (a) contacting the PFA biological sample with an array including a plurality of capture probes, where a capture probe of the plurality of capture probes includes (i) a spatial barcode and (ii) a capture domain, (b) decrosslinking one or more paraformaldehyde crosslinks in the PFA biological sample, such that capture domain specifically binds to the target nucleic acid, (c) extending the capture probe to generate a nucleic acid sequence that is complementary to a portion of the target nucleic acid, (d) generating a second strand hybridized to the extended capture probe, where the second strand includes a sequence complementary to the spatial barcode and the nucleic acid sequence corresponding to a portion of the sequence in the target nucleic acid, and (e) determining (i) all or a part of the sequence of the spatial barcode, or a complement thereof, and (ii) all or a part of the portion of the sequence of the target nucleic acid, or a complement thereof, and using the determined sequences of (i) and (ii) to identify the location of the target nucleic acid in the PFA biological sample.

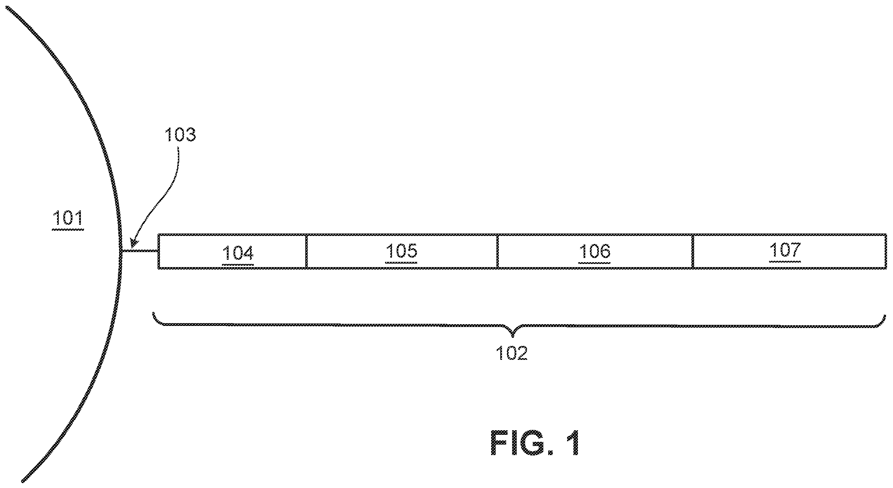

**FIG. 1** is a schematic diagram showing an example of a capture probe, as described herein. As shown, the capture probe **102** is optionally coupled to a feature **101** by a cleavable linker **103,** such as a photocleavable linker. The capture probe can include functional sequences that are useful for subsequent processing, such as functional sequence **104,** which can include a sequencer specific flow cell attachment sequence, e.g., a P5 or P7 sequence, as well as functional sequence **105,** which can include sequencing primer sequences, e.g., a R1 primer binding site, a R2 primer binding site. In some embodiments, sequence **104** is a P7 sequence and sequence **105** is a R2 primer binding site. A spatial barcode **106** can be included within the capture probe for use in barcoding the target analyte. The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **106,** functional sequences **104** (e.g., flow cell attachment sequence) and **105** (e.g., sequencing primer sequences) can be common to all of the probes attached to a given feature. The spatial barcode can also include a capture domain **107** to facilitate capture of a target analyte. Additional elements, such as unique molecular identifies, additional primer sites, etc. can be included, for example positioned between the spatial barcode **106** and the capture domain **107.**

### Decrosslinking

In some embodiments, the paraffin-embedding material can be removed (e.g., deparaffinization) from the biological sample (e.g., tissue section) by incubating the biological sample in an appropriate solvent (e.g., xylene), followed by a series of rinses (e.g., ethanol of varying concentrations), and rehydration in water. In some embodiments, the biological sample can be dried following deparaffinization. In some embodiments, after the step of drying the biological sample, the biological sample can be stained (e.g., H&E stain, any of the variety of stains described herein). In some embodiments, after staining the biological sample, the sample can be imaged.

After a fixed (e.g., FFPE, PFA, acetone, methanol) biological sample has undergone deparaffinization, the fixed (e.g., FFPE, PFA) biological sample can be further processed. For example, fixed (e.g., FFPE, PFA) biological samples can be treated to remove crosslinks (e.g., formaldehyde-induced crosslinks (e.g., decrosslinking)). In some embodiments, decrosslinking the crosslinks (e.g., formaldehyde-induced crosslinks) in the fixed (e.g., FFPE, PFA) biological sample can include treating the sample with heat. In some embodiments, decrosslinking the formaldehyde-induced crosslinks can include performing a chemical reaction. In some embodiments, decrosslinking the formaldehyde-induced crosslinks, can include treating the sample with a permeabilization reagent. In some embodiments, decrosslinking the formaldehyde-induced crosslinks can include heat, a chemical reaction, and/or permeabilization reagents.

In some embodiments, decrosslinking crosslinks (e.g., formaldehyde-induced crosslinks) can be performed in the presence of a buffer. In some embodiments, the buffer is Tris-EDTA (TE) buffer (e.g., TE buffer for FFPE biological samples). In some embodiments, the buffer is Tris-HCl buffer (e.g., Tris-HCl buffer for PFA fixed biological samples). In some embodiments, the buffer (e.g., TE buffer, Tris-HCl buffer) has a pH of about 7.0 to about 10.0, about 7.0 to about 9.9, about 7.0 to about 9.8, about 7.0 to about 9.7, about 7.0 to about 9.6, about 7.0 to about 9.5, about 7.0 to about 9.4, about 7.0 to about 9.3, about 7.0 to about 9.2, about 7.0 to about 9.1, about 7.0 to about 9.0, about 7.0 to about 8.9, about 7.0 to about 8.8, about 7.0 to about 8.7, about 7.0 to about 8.6, about 7.0 to about 8.5, about 7.0 to about 8.4, about 7.0 to about 8.3, about 7.0 to about 8.2, about 7.0 to about 8.1, about 7.0 to about 8.0, about 7.0 to about 7.9, about 7.0 to about 7.8, about 7.0 to about 7.7, about 7.0 to about 7.6, about 7.0 to about 7.5, about 7.0 to about 7.4, about 7.0 to about 7.3, about 7.0 to about 7.2, about 7.1 to about 10.0, about 7.2 to about 9.6, about 7.2 to about 9.5, about 7.2 to about 9.4, about 7.2 to about 9.3, about 7.2 to about 9.2, about 7.2 to about 9.1, about 7.2 to about 9.0, about 7.2 to about 8.9, about 7.2 to about 8.8, about 7.2 to about 8.7, about 7.2 to about 8.6, about 7.2 to about 8.5, about 7.2 to about 8.4, about 7.2 to about 8.3, about 7.2 to about 8.2, about 7.2 to about 8.1, about 7.2 to about 8.0, about 7.2 to about 7.9, about 7.2 to about 7.8, about 7.2 to about 7.7, about 7.2 to about 7.6, about 7.2 to about 7.5, about 7.2 to about 7.4, about 7.4 to about 10.0, about 7.4 to about 9.9, about 7.4 to about 9.8, about 7.4 to about 9.7, about 7.4 to about 9.6, about 7.4 to about 9.5, about 7.4 to about 9.4, about 7.4 to about 9.3, about 7.4 to about 9.2, about 7.4 to about 9.1, about 7.4 to about 9.0, about 7.4 to about 8.9, about 7.4 to about 8.8, about 7.4 to about 8.7, about 7.4 to about 8.6, about 7.4 to about 8.5, about 7.4 to about 8.4, about 7.4 to about 8.3, about 7.4 to about 8.2, about 7.4 to about 8.1, about 7.4 to about 8.0, about 7.4 to about 7.9, about 7.4 to about 7.8, about 7.4 to about 7.7, about 7.4 to about 7.6, about 7.6 to about 10.0, about 7.6 to about 9.9, about 7.6 to about 9.8, about 7.6 to about 9.7, about 7.6 to about 9.6, about 7.6 to about 9.5, about 7.6 to about 9.4, about 7.6 to about 9.3, about 7.6 to about 9.2, about 7.6 to about 9.1, about 7.6 to about 9.0, about 7.6 to about 8.9, about 7.6 to about 8.8, about 7.6 to about 8.7, about 7.6 to about 8.6, about 7.6 to about 8.5, about 7.6 to about 8.4, about 7.6 to about 8.3, about 7.6 to about 8.2, about 7.6 to about 8.1, about 7.6 to about 8.0, about 7.6 to about 7.9, about 7.6 to about 7.8, about 7.8 to about 10.0, about 7.8 to about 9.9, about 7.8 to about 9.8, about 7.8 to about 9.7, about 7.8 to about 9.6, about 7.8 to about 9.5, about 7.8 to about 9.4, about 7.8 to about 9.3, about 7.8 to about 9.2, about 7.8 to about 9.1, about 7.8 to about 9.0, about 7.8 to about 8.9, about 7.8 to about 8.8, about 7.8 to about 8.7, about 7.8 to about 8.6, about 7.8 to about 8.5, about 7.8 to about 8.4, about 7.8 to about 8.3, about 7.8 to about 8.2, about 7.8 to about 8.1, about 7.8 to about 8.0, about 7.9 to about 10.0, about 7.9 to about 9.9, about 7.9 to about 9.8, about 8.0 to about 10.0, about 8.0 to about 9.9, about 8.0 to about 9.8, about 8.0 to about 9.7, about 8.0 to about 9.6, about 8.0 to about 9.5, about 8.0 to about 9.4, about 8.0 to about 9.3, about 8.0 to about 9.2, about 8.0 to about 9.1, 8.0 to about 9.0, about 8.0 to about 8.9, about 8.0 to about 8.8, about 8.0 to about 8.7, about 8.0 to about 8.6, about 8.0 to about 8.5, about 8.0 to about 8.4, about 8.0 to about 8.3, about 8.0 to about 8.2, about 8.1 to about 10.0, about 8.2 to about 9.9, about 8.2 to about 9.8, about 8.2 to about 9.7, about 8.2 to about 9.6, about 8.2 to about 9.5, about 8.2 to about 9.4, about 8.2 to about 9.3, about 8.2 to about 9.2, about 8.2 to about 9.1, about 8.2 to about 9.0, about 8.2 to about 8.9, about 8.2 to about 8.8, about 8.2 to about 8.7, about 8.2 to about 8.6, about 8.2 to about 8.5, about 8.2 to about 8.4, about 8.4 to about 10.0, about 8.4 to about 9.9, about 8.4 to about 9.8, about 8.4 to about 9.7, about 8.4 to about 9.6, about 8.4 to about 9.5, about 8.4 to about 9.4, about 8.4 to about 9.3, about 8.4 to about 9.2, about 8.4 to about 9.1, about 8.4 to about 9.0, about 8.4 to about 8.9, about 8.4 to about 8.8, about 8.4 to about 8.7, about 8.4 to about 8.6, about 8.6 to about 10.0, about 8.6 to about 9.9, about 8.6 to about 9.8, about 8.6 to about 9.7, about 8.6 to about 9.6, about 8.6 to about 9.5, about 8.6 to about 9.4, about 8.6 to about 9.3, about 8.6 to about 9.2, about 8.6 to about 9.1, about 8.6 to about 9.0, about 8.6 to about 8.9, about 8.6 to about 8.8, about 8.8 to about 10.0, about 8.8 to about 9.9, about 8.8 to about 9.8, about 8.8 to about 9.7, about 8.8 to about 9.6, about 8.8 to about 9.5, about 8.8 to about 9.4, about 8.8 to about 9.3, about 8.8 to about 9.2, about 8.8 to about 9.1, about 8.8 to about 9.0, about 8.9 to about 10.0, about 8.9 to about 9.9, about 8.9 to about 9.8, about 8.9 to about 9.7, about 8.9 to about 9.6, about 8.9 to about 9.5, about 8.9 to about 9.4, about 8.9 to about 9.3, about 8.9 to about 9.2, or about 8.9 to about 9.1.

In some embodiments, the TE buffer (e.g., TE buffer for FFPE biological samples) has a temperature of about 60 °C to about 80 °C, between 60 °C to about 78 °C, about 60 °C to about 76 °C, about 60 °C to about 74 °C, about 60 °C to about 72 °C, about 60 °C to about 70 °C, about 60 °C to about 68 °C, about 60 °C to about 66 °C, about 60 °C to about 64 °C, about 60 °C to about 62 °C, about 62 °C to about 80 °C, between 62 °C to about 78 °C, about 62 °C to about 76 °C, about 62 °C to about 74 °C, about 62 °C to about 72 °C, about 62 °C to about 70 °C, about 62 °C to about 68 °C, about 62 °C to about 66 °C, about 62 °C to about 64 °C, about 64 °C to about 80 °C, between 64 °C to about 78 °C, about 64 °C to about 76 °C, about 64 °C to about 74 °C, about 64 °C to about 72 °C, about 64 °C to about 70 °C, about 64 °C to about 68 °C, about 64 °C to about 66 °C, about 66 °C to about 80 °C, between 66 °C to about 78 °C, about 66 °C to about 76 °C, about 66 °C to about 74 °C, about 66 °C to about 72 °C, about 66 °C to about 70 °C, about 66 °C to about 68 °C, about 68 °C to about 80 °C, between 68 °C to about 78 °C, about 68 °C to about 76 °C, about 68 °C to about 74 °C, about 68 °C to about 72 °C, about 68 °C to about 70 °C, about 70 °C to about 80 °C, between 70 °C to about 78 °C, about 70 °C to about 76 °C, about 70 °C to about 74 °C, about 70 °C to about 72 °C, about 72 °C to about 80 °C, between 72 °C to about 78 °C, about 72 °C to about 76 °C, about 72 °C to about 74 °C, about 74 °C to about 80 °C, between 74 °C to about 78 °C, about 74 °C to about 76 °C, about 76 °C to about 80 °C, between 76 °C to about 78 °C, or about 78 °C to about 80 °C.

In some embodiments, the Tris-HCl buffer (e.g., Tris-HCl buffer for PFA fixed biological samples) has a temperate of about 50°C to about 70°C, about 50°C to about 68°C, about 50° to about 66°C, about 50°C to about 64°, about 50° to about 62°C, about 50°C to about 60°C, about 50°C to about 58°C, about 50°C to about 56°C, about 50°C to about 54°C, about 50°C to about 52°C, about 52°C to about 70°C, about 52°C to about 68°C, about 52°C to about 66°C, about 52°C to about 64°C, about 52°C to about 62°, about 52°C to about 60°C, about 52°C to about 58°C, about 52°C to about 56°C, about 52°C to about 54°C, about 54°C to about 70°C, about 54°C to about 68°C, about 54°C to about 66°C, about 54°C to about 64°C, about 54°C to about 62°C, about 54°C to about 60°C, about 54°C to about 58°C, about 54°C to about 56°, about 56°C to about 70°C, about 56°C to about 68°C, about 56°C to about 66°C, about 56°C to about 64°C, about 56°C to about 62°C, about 56°C to about 60°, about 56°C to about 58°C, about 58°C to about 70°C, about 58°C to about 68°C, about 58°C to about 66°C, about 58°C to about 64°C, about 58°C to about 62°C, about 58°C to about 60°C, about 60°C to about 70°C, about 60°C to about 68°C, about 60°C to about 66°C, about 60°C to about 64°C, about 60°C to about 62°C, about 62°C to about 70°C, about 62°C to about 68°C, about 62°C to about 66°C, about 62°C to about 64°C, about 64°C to about 70°C, about 64°C to about 68°C, about 64°C to about 66°C, about 66°C to about 70°C, about 66°C to about 68°C, or about 68°C to about 70°C.

In some embodiments, the fixed biological sample can be contacted with a buffer. In some embodiments, a PFA fixed biological sample can be contacted with Tris-HCl buffer. In some embodiments, a FFPE biological sample can be contacted with TE buffer. In some embodiments, the FFPE biological sample can be contacted with TE buffer and the PFA fixed biological sample can be contacted with Tris-HCl buffer for about 10 minutes to about 200 minutes, about 10 minutes to about 190 minutes, about 10 minutes to about 180 minutes, about 10 minutes to about 170 minutes, about 10 minutes to about 160 minutes, about 10 minutes to about 150 minutes, about 10 minutes to about 140 minutes, about 10 minutes to about 130 minutes, about 10 minutes to about 120 minutes, about 10 minutes to about 110 minutes, about 10 minutes to about 100 minutes, about 10 minutes to about 90 minutes, about 10 minutes to about 80 minutes, about 10 minutes to about 70 minutes, about 10 minutes to about 65 minutes, about 10 minutes to about 60 minutes, about 10 minutes to about 55 minutes, about 10 minutes to about 50 minutes, about 10 minutes to about 40 minutes, about 10 minutes to about 30 minutes, about 10 minutes to about 20 minutes, about 20 minutes to about 120 minutes, about 20 minutes to about 110 minutes, about 20 minutes to about 100 minutes, about 20 minutes to about 90 minutes, about 20 minutes to about 80 minutes, about 20 minutes to about 70 minutes, about 20 minutes to about 65 minutes, about 20 minutes to about 60 minutes, about 20 minutes to about 55 minutes, about 20 minutes to about 50 minutes, about 20 minutes to about 40 minutes, about 20 minutes to about 30 minutes, about 30 minutes to about 120 minutes, about 30 minutes to about 110 minutes, about 30 minutes to about 100 minutes, about 30 minutes to about 90 minutes, about 30 minutes to about 80 minutes, about 30 minutes to about 70 minutes, about 30 minutes to about 65 minutes, about 30 minutes to about 60 minutes, about 30 minutes to about 55 minutes, about 30 minutes to about 50 minutes, about 30 minutes to about 40 minutes, about 40 minutes to about 120 minutes, about 40 minutes to about 110 minutes, about 40 minutes to about 100 minutes, about 40 minutes to about 90 minutes, about 40 minutes to about 80 minutes, about 40 minutes to about 70 minutes, about 40 minutes to about 65 minutes, about 40 minutes to about 60 minutes, about 40 minutes to about 55 minutes, about 40 minutes to about 50 minutes, about 50 minutes to about 120 minutes, about 50 minutes to about 110 minutes, about 50 minutes to about 100 minutes, about 50 minutes to about 90 minutes, about 50 minutes to about 80 minutes, about 50 minutes to about 70 minutes, about 50 minutes to about 65 minutes, about 50 minutes to about 60 minutes, about 50 minutes to about 55 minutes, about 55 minutes to about 120 minutes, about 55 minutes to about 110 minutes, about 55 minutes to about 100 minutes, about 55 minutes to about 90 minutes, about 55 minutes to about 80 minutes, about 55 minutes to about 70 minutes, about 55 minutes to about 65 minutes, about 55 minutes to about 60 minutes, about 60 minutes to about 200 minutes, about 60 minutes to about 120 minutes, about 60 minutes to about 110 minutes, about 60 minutes to about 100 minutes, about 60 minutes to about 90 minutes, about 60 minutes to about 80 minutes, about 60 minutes to about 70 minutes, about 60 minutes to about 65 minutes, about 65 minutes to about 120 minutes, about 65 minutes to about 110 minutes, about 65 minutes to about 100 minutes, about 65 minutes to about 90 minutes, about 65 minutes to about 80 minutes, about 65 minutes to about 70 minutes, about 70 minutes to about 200 minutes, about 70 minutes to about 190 minutes, about 70 minutes to about 180 minutes, about 70 minutes to about 170 minutes, about 70 minutes to about 160 minutes, about 70 minutes to about 150 minutes, about 70 minutes to about 140 minutes, about 70 minutes to about 130 minutes, or about 70 minutes to about 120 minutes.

In some embodiments, the FFPE biological sample can be contacted with TE buffer that has a temperature of about 65°C to about 75°C, and is contacted with the FFPE biological sample for about 30 minutes to about 90 minutes. In some embodiments, the TE buffer can have a temperature of about 70 °C, a pH of about 8.0, and can be contacted with the FFPE biological sample for about 60 minutes.

In some embodiments, the PFA fixed biological sample can be contacted with Tris-HCl buffer that has a temperature of about 55°C to about 65°C, a pH of about 8.5 to 9.5, and is contacted with the PFA fixed sample for about 30-90 minutes. In some embodiments, the Tris-HCl buffer has a temperature of about 60°C, a pH of about 9.0, and is contacted with the PFA fixed biological sample for 60 minutes.

After decrosslinking the crosslinks (e.g., decrosslinking formaldehyde or paraformaldehyde crosslinks) in the FFPE biological sample (e.g., FFPE tissue section), the biological sample can be permeabilized (e.g., permeabilized by any of the variety of methods described herein). In some embodiments, the fixed (e.g., FFPE, PFA) biological sample can be permeabilized with a protease. In some embodiments, the protease can be pepsin. In some embodiments, the protease can be proteinase K. In some embodiments, the protease can be pepsin and proteinase K. In some embodiments, the fixed (e.g., FFPE, PFA) biological sample can be permeabilized with a protease for about 10 minutes to about 90 minutes (e.g., about 10 minutes to about 80 minutes, about 10 minutes to about 70 minutes, about 10 minutes to about 60 minutes, about 10 minutes to about 50 minutes, about 10 minutes to about 40 minutes, about 10 minutes to about 30 minutes, about 10 minutes to about 20 minutes, about 20 minutes to about 90 minutes, about 20 minutes to about 80 minutes, about 20 minutes to about 70 minutes, about 20 minutes to about 60 minutes, about 20 minutes to about 50 minutes, about 20 minutes to about 40 minutes, about 20 minutes to about 30 minutes, about 30 minutes to about 90 minutes, about 30 minutes to about 80 minutes, about 30 minutes to about 70 minutes, about 30 minutes to about 60 minutes, about 30 minutes to about 50 minutes, about 30 minutes to about 40 minutes, about 40 minutes to about 90 minutes, about 40 minutes to about 80 minutes, about 40 minutes to about 70 minutes, about 40 minutes to about 60 minutes, about 40 minutes to about 50 minutes, about 50 minutes to about 90 minutes, about 50 minutes to about 80 minutes, about 50 minutes to about 70 minutes, or about 50 minutes to about 60 minutes).

Permeabilization of a fixed (e.g., FFPE, PFA) biological sample preferably occurs on a substrate, for example a substrate including a plurality of capture probes (e.g., an array). For example, the fixed (e.g., FFPE, PFA) biological sample can be applied to any of the variety of arrays described herein. In some embodiments, additional capture probes of the plurality of capture probes include in a 5' to a 3' direction, a spatial barcode and a poly(T) capture domain. For example, the plurality of capture probes can include one or more capture probes with a random nucleic acid capture domain and one or more capture probes with a poly(T) capture domain. In some embodiments, a capture probe can include one or more functional domains and/or a cleavage domain. A functional domain typically includes a functional nucleotide sequence for a downstream analytical step in the overall analysis procedure. In some embodiments, the functional domain can include a sequencing handle. In some embodiments, the functional domain can include a PCR handle. In some embodiments, a capture probe can include a unique molecular identifier described herein. In some embodiments, the unique molecular identifier is located 5' to the capture domain in the capture probe. In some embodiments, the poly(T) capture domain can capture a target nucleic acid. In some embodiments, the target nucleic acid is DNA. In some embodiments, the target nucleic is RNA (e.g., any of the RNA analytes described herein). In some embodiments, the RNA is mRNA.

In some embodiments, the fixed (e.g., FFPE, PFA) biological sample can be incubated with an exonuclease prior to reverse transcription (e.g., extension of the 3' end of the capture probe) of the analyte (e.g., an analyte bound to the capture probe). Free single-stranded DNA molecules present in the biological sample can be digested to reduce capture of single-stranded DNA molecules by the capture probes. For example, the fixed (e.g., FFPE, PFA) biological sample can be incubated with a 5' to 3' single-stranded DNA exonuclease prior to reverse transcription of an analyte (e.g., an analyte bound to a capture probe). The capture probes are bound to the substrate or feature by their 5' end and thus are protected from digestion by a 5' to 3' exonuclease. In some embodiments, the 5' to 3' exonuclease is T7 exonuclease. In some embodiments, the 5' to 3' exonuclease is RecJ exonuclease. Alternatively or additionally, the fixed (e.g., FFPE, PFA) biological sample can be incubated with a double-stranded DNA endonuclease prior to reverse transcription. In some embodiments, the fixed (e.g., FFPE, PFA) biological sample can be incubated with both a 5' to 3' exonuclease and a double-stranded DNA endonuclease.

In some embodiments, after reverse transcription (e.g., extension of the 3' end of the capture probe) of the analyte (e.g., analyte bound to the capture probe), the extended capture probe can be contacted with a labeled (e.g., labeled with any of the detectable labels described herein) nucleic acid substantially complementary to the extended capture probe, or a portion thereof. In some embodiments, the labeled nucleic acid can bind (e.g., hybridize) to the extended capture probe. In some embodiments, the labeled nucleic acid (e.g., labeled with a detectable label) can be detected to identify the location of the target nucleic acid in the fixed (e.g., FFPE, PFA) biological sample.

### Second-strand synthesis

In some embodiments, reverse transcription of an analyte (e.g., an analyte bound to a capture probe) can be performed in the presence of actinomycin D. Actinomycin D can specifically inhibit DNA-dependent DNA polymerase activity of a reverse transcriptase enzyme. Reverse transcription can be performed with any of the variety of reverse transcriptase enzymes described herein and by any of the methods described herein. In some embodiments, during reverse transcription the reverse transcriptase enzyme can add a homonucleotide sequence to the 3' end of the extended capture probe. In some embodiments, during reverse transcription (e.g., extending the capture probe) the reverse transcriptase enzyme can add a poly(C) sequence to the 3' end of the extended capture probe. In some embodiments, the poly(C) sequence can be added by a terminal transferase enzyme (e.g., TdT) in the presence of dCTP. In some embodiments, the terminal transferase enzyme can be terminal deoxynucleotidyl transferase. In some embodiments, the poly(C) sequence can be added after removal of the FFPE biological sample.

In some embodiments, a template switching oligonucleotide (e.g., a template switching oligonucleotide described herein) complementary to the added poly(C) sequence can be provided to the FFPE biological sample. In some embodiments, a functional domain (e.g., sequencing or PCR handle) can be ligated to the 3' end of the additional poly(C) sequence. In some embodiments, the sequencing handle can be ligated to the 3' end of the additional poly(C) sequence (e.g., poly(C) sequence added to the capture probe) with a splint oligonucleotide. The ligase can be any ligase described herein. In some embodiments, the ligase is T4 DNA ligase (Gansaunge, M.T., Single-stranded DNA library preparation from highly degraded DNA using T4 DNA ligase, Nucleic Acids Research, 45, 10 e79 doi: 10.1093narlgkx033 (2017)). For example, a partially double stranded splint oligonucleotide can include a first oligonucleotide with a functional domain (e.g., a sequencing or PCR handle) and a second oligonucleotide that includes in the 5' to 3' direction: a poly(G) sequence that specifically binds to the added poly(C) sequence of the capture probe and a single stranded portion substantially complementary to the functional domain (e.g., sequencing handle). In some embodiments, the second oligonucleotide of the partially double stranded splint oligonucleotide can have a 5' overhang (e.g., single stranded portion) that includes at least a portion of the poly(G) sequence. In some embodiments, a functional domain (e.g., sequencing or PCR handle) can be added to the 3' end of the additional homonucleotide sequence (e.g., poly(C) sequence) by a DNA polymerase extension reaction.

In some embodiments, after ligation of the functional domain (e.g., sequencing or PCR handle) to the 3' end of the capture probe (e.g., ligating the sequencing handle to the additional poly(C) sequence) or the addition of the functional domain by a DNA polymerase extension reaction, the second oligonucleotide of the splint oligonucleotide can be dissociated from the capture probe with the ligated functional domain (e.g., sequencing or PCR handle). In some embodiments, the second oligonucleotide can be dissociated with potassium hydroxide (KOH). In some embodiments, a primer substantially complementary to the sequencing handle of the capture probe can be hybridized to the sequencing handle and extended to generate a second strand.

In some embodiments, after reverse transcription generating a second strand hybridized to the extended capture probe that includes a sequence complementary to the spatial barcode and the sequence, or a portion thereof, of a target nucleic acid, an adaptor can be ligated directly to the 3' end of the capture probe (e.g., extended capture probe). In some embodiments, the adaptor can include a functional domain (e.g., sequencing or PCR handle). In some embodiments, a primer substantially complementary to the functional domain of the adaptor can be hybridized to the sequencing handle and extended to generate a second strand.

In some embodiments, after reverse transcription generating a second strand hybridized to the extended capture probe that includes a sequence complementary to the spatial barcode and the sequence, or a portion thereof, of a target nucleic acid, an adaptor (e.g., a 5' adenylated adaptor) can be attached to the 3' end of the capture probe (e.g., extended capture probe). In some embodiments, the adapter is a 5' adenylated adaptor. In some embodiments, the adaptor can contain a 5' phosphate. In some embodiments, the 5' adenylated adaptor can be attached with a ligase. In some embodiments the ligase is a single-stranded RNA ligase. In some embodiments, the ligase is a single-stranded DNA ligase. In some embodiments, the ligase is T4 RNA ligase 2. In some embodiments, the ligase is 5' App DNA/RNA ligase (See, Hafner, M., et. al., Identification of microRNAs and other small regulatory RNAs using cDNA library sequencing, Methods 44(1): 3-12 doi: 10.1016/j.ymeth.2007.09.009 (2008)). In some embodiments, the 5' adenylated adaptor can include a functional domain (e.g., a sequencing or PCR handle). In some embodiments, a primer substantially complementary to the functional domain in the 5' adenylated adapter can be hybridized to the functional domain (e.g., sequencing or PCR handle) and extended to generate a second strand.

In some embodiments, after reverse transcription generating a second strand hybridized to the extended capture probe that includes a sequence complementary to the spatial barcode and the sequence, or a portion thereof, of a target nucleic acid, a poly(A) sequence can be added to the end of the capture probe (e.g., extended capture probe) and an adaptor can be ligated to the 3' end of the poly(A) sequence in the capture probe (e.g., TdT-assisted adenylate connector-mediated single-stranded DNA ligation (TACS ligation)). In some embodiments, the poly(A) sequence can be a ribonucleic acid sequence. In some embodiments, the poly(A) sequence can be added to the 3' end of the capture probe with a terminal transferase (e.g., terminal deoxynucleotidyl transferase) in the presence of ATP (Boule, J. et. al., Terminal Deoxynucleotidyl Transferase Indiscriminately Incorporates Ribonucleotides and Deoxyribonucleotides, The Journal of Biological Chemistry, 276, 31388-31393, doi: 10.1074/jbc.M105272200 (2001)). For example, the capture probe (e.g., extended capture probe) includes DNA (e.g., cDNA generated by reverse transcription) followed by an RNA sequence (e.g., poly(A) RNA sequence). In some embodiments, ligating the adaptor to the ribonucleic poly(A) sequence can be performed with an RNA ligase (Miura, F. et. al., Highly efficient single-stranded DNA ligation technique improves low-input whole-genome bisulfite sequencing by post-bisulfite adaptor tagging, Nucleic Acids Research, 47, 15 e85 doi: 10.1093/narlgkz435 (2019)). In some embodiments, ligating the adaptor to the poly(A) sequence can be performed using CircLigase. In some embodiments, the adaptor can include a functional domain (e.g., a sequencing or PCR handle). In some embodiments, a primer substantially complementary to the functional domain (e.g., sequencing or PCR handle) in the adaptor can be hybridized to the functional domain (e.g., sequencing or PCR handle) and extended to generate a second strand. In some embodiments, extending the primer to generate the second strand can be performed with a DNA polymerase capable of processing RNA bases as a substrate. In some embodiments, the DNA polymerase can be a Taq polymerase. In some embodiments, the Taq polymerase is a derivative or modified Taq-polymerase.

In some embodiments, after reverse transcription, a second strand hybridized to the extended capture probe is generated that includes a sequence complementary to the spatial barcode and the sequence, or a portion thereof, of a target nucleic acid. In some embodiments, the second strand is generated through hybridizing one or more primers that include a random sequence and a functional domain. In some embodiments, hybridizing one or more primers that include a random sequence and a functional domain can be performed after the fixed biological sample has been removed from the array. In some embodiments, the functional domain includes a template switching oligonucleotide (TSO) sequence followed by a random sequence. In some embodiments, the functional domain is followed by a random sequence comprising SEQ ID NO: 4. In some embodiments, the functional domain is followed by a random sequence comprising SEQ NO: 5. In some embodiments, a primer including a functional domain followed by a random sequence (e.g., any of the primers described herein) can generate a second strand in combination with a template switching oligonucleotide. For example, a sequence complementary to a TSO can be incorporated into an extended capture probe during reverse transcription and a combination of TSO primers and primers including a functional domain followed by a random sequence can be used to generate a second strand.

In some embodiments, the random sequence can hybridize to a sequence in the capture probe (e.g., extended capture probe) that is substantially complementary to the random sequence. In some embodiments, the random sequence can be about 6 nucleotides, about 7 nucleotides, about 8 nucleotides, about 9 nucleotides, or about 10 nucleotides. In some embodiments, the functional domain can include a sequencing handle. In some embodiments, the functional domain can include a PCR handle. In some embodiments, the one or more probes hybridized the capture probe (e.g., extended capture probe) can be extended to generate a second strand (Hughes, T.K., Highly Efficient, Massively-Parallel Single-Cell RNA-Seq Reveals Cellular States and Molecule Features of Human Skin Pathology, doi: https://doi.org/10.1101/689273 (2019)). In some embodiments, the one or more probes hybridized to the capture probe can be extended with a strand displacing polymerase. In some embodiments, the strand displacing polymerase can be Klenow fragment.

In some embodiments, generating the second strand can be performed at temperature of about 30°C to about 40°C. In some embodiments, generating the second strand can be performed at a temperature of about 31°C, about 32°C, about 33°C, about 34°C, about 35°C, about 36°C, about 37°C, about 38°C, or about 39°C.

In some embodiments, the FFPE biological sample can be treated with one or more restriction endonucleases after reverse transcription is performed. The process of reverse transcription extends the 3' end of the capture probe using the captured target nucleic acid as a template. In some embodiments, capture probes containing random nucleic acid capture domains can non-specifically capture nucleic acids (e.g., RNA and/or DNA). In some embodiments, after reverse transcription, the extended capture probe can contain DNA-RNA (e.g., captured RNA) double stranded molecules and DNA-DNA (e.g., captured DNA) double stranded molecules. In some embodiments, after the reverse transcription reaction, the DNA-DNA double-stranded molecules resulting from non-specifically captured nucleic acids (e.g., genomic DNA) can be digested. In some embodiments, the DNA-DNA double-stranded molecules can be digested (e.g., fragmented) with one or more restriction endonucleases that have an AT-rich restriction endonuclease recognition sequence. For example, some non-limiting examples of restriction endonucleases with AT-rich recognition sequences include, HindIII, Sspl, EcoR1, and MfeI.

In some embodiments, the fixed (e.g., FFPE, PFA) biological sample can be an FFPE tissue section. In some embodiments, the fixed biological sample is a PFA fixed tissue section. In some embodiments, the fixed (e.g., FFPE, PFA) biological sample can be imaged (e.g., imaged by any of the methods described herein).

In another aspect provided herein, the location of a target nucleic acid in a fixed (e.g., FFPE, PFA) biological sample can be identified using ligation via in situ hybridization. For example, the method can include contacting the FFPE biological sample with a first probe and a second probe. In some embodiments, the first probe can have in a 5' to a 3' direction: a first functional domain and a first sequence that is substantially complementary to a portion of the target nucleic acid. In some embodiments, the first probe can have a 3' end that includes a 3' diribo sequence. In some embodiments, the first probe can have a 3' end that includes a 3' deoxynucleotide sequence. For example, the first probe can be a DNA/RNA hybrid probe. In some embodiments, the first probe can be a DNA probe. In some embodiments, the second probe can have in a 5' to a 3' direction: a second sequence that is substantially complementary to a portion of the target nucleic acid and an analyte capture sequence. In some embodiments, the analyte capture sequence can be a sequence complementary to a capture domain (e.g., any of the capture domains described herein) of a capture probe. In some embodiments, the second probe can have a 5' end phosphate. In some embodiments, the first sequence and the second sequence can bind specifically to the target nucleic acid (e.g., mRNA).

In some embodiments, the 3' end of the first probe can be ligated to the 5' end of the second probe thereby generating a ligated product. In some embodiments, the first probe and the second probe can be ligated with a ligase. In some embodiments, the ligase can be any ligase capable of ligating RNA and DNA together. In some embodiments, the ligase can be any DNA ligase. In some embodiments, the ligase can be T4 RNA ligase 2. In some embodiments, prior to contacting the fixed (e.g., FFPE, PFA) biological sample with an array and after ligation of the first probe and the second probe, the fixed (e.g., FFPE, PFA) biological sample can be treated with an RNase.

In some embodiments, the fixed (e.g., FFPE, PFA) biological sample can be contacted with an array that includes a plurality of capture probes. In some embodiments, a capture probe of the plurality of capture probes can have in a 5' to a 3' direction: a spatial barcode and a capture domain. In some embodiments, the capture probe can also include a cleavage domain, a unique molecular identifier, one or more functional domains, or combinations thereof. In some embodiments, the capture domain can have a sequence substantially complementary to the analyte capture sequence. In some embodiments, the capture domain can specifically bind to the analyte capture sequence domain of the ligated product.

In some embodiments, the unligated probes (e.g., unligated first probes and unligated second probes) can be removed or blocked from binding the capture probes of the array. For example, the unligated second probe including the analyte capture sequence (e.g., the sequence complementary to the capture domain) can bind (e.g., hybridize) to the capture domain (e.g., any of the capture domains described herein). In some embodiments, when the unligated second probe binds to the capture domain it can block ligated probes from binding (e.g., hybridizing) with the capture domain. In some embodiments, the first probe can non-specifically bind sequences in the capture probe (e.g., spatial barcode, unique molecular identifier, etc.).

In some embodiments, capture domains of the capture probes on the array can be blocked to prevent unligated second probes from binding. In some embodiments, after blocking capture sites of the capture domain and/or ligated probe an exonuclease can be added to the biological sample. In some embodiments, the exonuclease can be RecJε. In some embodiments, the exonuclease can be a 5' to 3' riboexonuclease. In some embodiments, the riboexonuclease can be Terminator^{™} nuclease. In some embodiments, the exonuclease can be a combination of RecJε and Terminator^{™} nuclease. In some embodiments, an endonuclease can be added to the fixed (e.g., FFPE, PFA) biological sample. In some embodiments, the endonuclease is RNase HI or RNase HII. In some embodiments, the endonuclease can nick unligated first probes non-specifically bound (e.g., hybridized) to the capture probe, or portion thereof.

In some embodiments, the 3' end of the capture probe can be extended to add a sequence that corresponds (e.g., complementary) to a portion of the target nucleic acid, the spatial barcode, the unique molecular identifier, one or more functional domains, and the analyte capture sequence. In some embodiments, a second strand can be generated (e.g., generate a second strand sequence complementary to the capture probe (e.g., extended capture probe)). In some embodiments, the second strand can include a sequence complementary to the spatial barcode and a sequence reverse complementary to a portion of the target nucleic acid. In some embodiments, the ligation products (e.g., the ligated first probe and second probe) are removed (e.g., melted away with increased temperature) and the extended capture probe can be used to generate a sequencing library. In some embodiments, after the ligated product is removed, second strand synthesis can be performed on the extended capture probe (e.g., by any of the methods described herein). The generated second strand can be removed (e.g., melted away with increased temperature) and used to generate a sequencing library.

In some embodiments, the sequence of all, or a part of, the spatial barcode or complement thereof and the sequence of all, or a part of, the target nucleic acid or complement thereof can be determined. In some embodiments, the determined sequences can be used to identify the location of the target nucleic acid in the fixed (e.g., FFPE, PFA) biological sample. In some embodiments, determining the sequences can be performed by any of the sequencing methods described herein.

In some embodiments, the first functional domain can be a first sequencing handle. In some embodiments, the analyte capture sequence a sequence substantially complementary to the capture domain, or portion thereof.

In some embodiments, a primer can be hybridized to at least a portion of the first functional domain to the capture probe.

In some embodiments, prior to contacting the fixed (e.g., FFPE, PFA) biological sample with the first probe and the second probe, the formaldehyde crosslinks in the FFPE biological sample can be decrosslinked (e.g., decrosslinked by any of the methods described herein). For example, the formaldehyde crosslinks can be decrosslinked with heat, performing a chemical reaction, through the use of an enzyme, and/or a buffer (e.g., TE buffer for FFPE samples, Tris-HCl buffer for PFA fixed samples). In some embodiments, the crosslinks (e.g., formaldehyde crosslinks) can be decrosslinked with the use of a buffer (e.g., TE buffer, Tris-HCl buffer) under any of the conditions described herein. In some embodiments, the fixed (e.g., FFPE, PFA) biological sample can be permeabilized, after decrosslinking the formaldehyde crosslinks by any of the methods described herein (e.g., permeabilization with a protease such as pepsin and/or proteinase K).

Methods and compositions provided herein are further described in the following examples.

### Spatial Analysis in Poor Quality Biological Samples

Nucleic acids present in a biological sample can often be degraded or fragmented in a poor quality biological sample. For example, DNA and/or RNA can be degraded or fragmented upon tissue fixation such as when a tissue is fixed for pathological study by FFPE methodologies. Molecularly, DNA and/or RNA degradation or fragmentation can result from a number of factors; the timing between tissue acquisition and fixation, cellular processes and autolysis, high temperature incubation of tissues during fixation (e.g., during embedding processes), prolonged storage of fixed tissues, or staining processes when tissues are stained. The degradation or fragmentation of DNA and/or RNA can greatly affect the efficiency of capture of DNA and/or RNA sequences for spatial array assays.

### Adding an Analyte Capture Sequence

As an example, when mRNA is fragmented some of the mRNA fragments are polyadenylated whereas other mRNA fragments would not be polyadenylated. However, mRNA fragments that are not polyadenylated are still important to capture for analysis. In some embodiments, an analyte capture sequence can be added to analytes (e.g., DNA and/or RNA) that are missing a capture sequence, such as fragments of mRNA that lack a polyadenylated sequence. As used herein, an "analyte capture sequence" includes a sequence that is substantially complementary to a capture domain of a capture probe. In some embodiments, the analyte capture sequence can include a sequence that is at least 80%, at least 82%, at least 84%, at least 86%, at least 88%, at least 90%, at least 92%, at least 94%, at least 96%, at least 98%, at least 99%, or 100% complementary to a capture domain (e.g., any of the exemplary capture domains described herein).

In some embodiments, the analyte capture sequence can include a homonucleotide sequence. In other embodiments, the analyte capture sequence can include a heteronucleotide sequence. In some embodiments, an analyte capture sequence (e.g., a homonucleotide sequence or a heteronucleotide sequence) can be added to analytes present in a biological sample. In some embodiments, an analyte capture sequence (e.g., a homonucleotide sequence or a heteronucleotide sequence) can be added to DNA (e.g., genomic DNA). In some embodiments, the genomic DNA includes a mutation or a single nucleotide polymorphism.

In some embodiments, an analyte capture sequence (e.g., a homonucleotide sequence or heteronucleotide sequence) can be added to RNA (e.g., mRNA, miRNA, siRNA, snRNA, and tRNA) (Slomovic, S., et al., Addition of poly(A) and poly(A)-rich tails during RNA degradation in the cytoplasm of human cells, PNAS, 107(16), 7407-7412, (2010)). In some embodiments, the mRNA includes a single nucleotide polymorphism or a mutation. In some embodiments, the mRNA includes a splice variant. In some embodiments, multiple types of analytes (e.g., at least two, at least three, at least four different types of analytes) can be captured. In some embodiments, addition of an analyte capture sequence (e.g., a homonucleotide sequence or heteronucleotide sequence) can be added to an RNA species (e.g., miRNA) that would not otherwise be captured by a poly(T) or poly(A) capture domain of a capture probe of the array.

In some embodiments, the homonucleotide sequence can be a poly(A) sequence or a poly(T) sequence. In some embodiments, the heteronucleotide sequence can be a mix of nucleotides. For example, a homonucleotide sequence such as a poly(A) tail can be added to RNA (e.g., mRNA) in the biological sample. In some embodiments, a poly(A) tail can be added to DNA (e.g., genomic DNA) in the biological sample. In some embodiments, the poly(A) tail can be added to a nucleic acid analyte (e.g., DNA or RNA) present in the biological sample with a poly(A) polymerase in the presence of buffers and dATP. In some embodiments, the poly(A) tail can be added to a nucleic acid analyte (e.g., DNA or RNA) present in the biological sample with a poly(A) polymerase in the presence of buffers and oligo-d(A)s. In some embodiments, an analyte capture sequence (e.g., a poly(A) tail) can be added to RNA present in a poor quality biological sample with a template-independent polymerase (e.g., TdT). In some embodiments, an analyte capture sequence (e.g., a heteronucleotide sequence) can be added to RNA present in a poor quality biological sample with a template-independent polymerase (e.g., TdT). In some embodiments, an analyte capture sequence (e.g., a homonucleotide sequence or a heteronucleotide sequence) can be added to the 3' end of the analyte (e.g., RNA or DNA) in the biological sample. In some embodiments, the added homonucleotide sequence (e.g., a poly(A) or poly(T) sequence) or heteronucleotide sequence can have a total of about 1 to about 50 nucleotides (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides, or about 1 to about 45 nucleotides, about 1 to about 40 nucleotides, about 1 to about 35 nucleotides, about 1 to about 30 nucleotides, about 1 to about 25 nucleotides, about 1 to about 20 nucleotides, about 1 to about 15 nucleotides, about 1 to about 10 nucleotides, about 1 to about 5 nucleotides, about 5 to about 50 nucleotides, about 5 to about 45 nucleotides, about 5 to about 40 nucleotides, about 5 to about 35 nucleotides, about 5 to about 30 nucleotides, about 5 to about 25 nucleotides, about 5 to about 20 nucleotides, about 5 to about 15 nucleotides, about 5 to about 10 nucleotides, about 10 to about 50 nucleotides, about 10 to about 45 nucleotides, about 10 to about 40 nucleotides, about 10 to about 35 nucleotides, about 10 to about 30 nucleotides, about 10 to about 25 nucleotides, about 10 to about 20 nucleotides, about 10 to about 15 nucleotides, about 15 to about 50 nucleotides, about 15 to about 45 nucleotides, about 15 to about 40 nucleotides, about 15 to about 35 nucleotides, about 15 to about 30 nucleotides, about 15 to about 25 nucleotides, about 15 to about 20 nucleotides, about 20 to about 50 nucleotides, about 20 to about 45 nucleotides, about 20 to about 40 nucleotides, about 20 to about 35 nucleotides, about 20 to about 30 nucleotides, about 20 to about 25 nucleotides, about 25 to about 50 nucleotides, about 25 to about 45 nucleotides, about 25 to about 40 nucleotides, about 25 to about 35 nucleotides, about 25 to about 30 nucleotides, about 30 to about 50 nucleotides, about 30 to about 45 nucleotides, about 30 to about 40 nucleotides, about 30 to about 35 nucleotides, about 35 to about 50 nucleotides, about 35 to about 45 nucleotides, about 35 to about 40 nucleotides, about 40 to about 50 nucleotides, about 40 to about 45 nucleotides, or about 45 to about 50 nucleotides).

In some embodiments, an analyte capture sequence (e.g., a homopolynucleotide sequence or a heteropolynucleotide sequence) can be ligated to a nucleic acid analyte (e.g., DNA or RNA) present in the biological sample. In some embodiments, the analyte capture sequence (e.g., a homopolynucleotide sequence or heteropolynucleotide) can be ligated with a ligase (e.g., any ligase described herein). In some embodiments, the ligase is an RNA ligase (e.g., T4 ligase). In some embodiments, the ligase is a DNA ligase.

In some embodiments, after an analyte capture sequence (e.g., poly(A) sequence, poly(T) sequence, heteronucleotide sequence, homopolynucleotide sequence, heteropolynucleotide sequence) is added to a nucleic acid analyte present in the poor quality biological sample (e.g., via use of a polymerase or via ligation), the poor quality biological sample can be contacted with an array including capture probes. In some embodiments, the added analyte capture sequence (e.g., poly(A) sequence, poly(T) sequence, heteronucleotide sequence) can specifically bind (e.g., hybridize) the capture domain of a capture probe of the array. In some embodiments, reverse transcription can be performed to extend the 3' end of the capture probe using the captured analyte as a template. In some embodiments, a second strand hybridized to the extended capture probe can be generated (e.g., generated by any of the methods described herein), where the second strand includes a sequence complementary to the spatial barcode and at least a portion of the sequence of the analyte. Some embodiments of any of the methods described herein include releasing the second strand from the extended capture probe.

In some embodiments, after an analyte capture sequence (e.g., poly(A) sequence, poly(T) sequence, heteronucleotide sequence) is added to a nucleic acid analyte present in the poor quality biological sample (e.g., via use of a polymerase or via ligation), the poor quality biological sample can be contacted with an array including capture probes. In some embodiments, the added analyte capture sequence (e.g., poly(A) sequence, poly(T) sequence, heteronucleotide sequence) can specifically bind (e.g., hybridize) the capture domain of a capture probe of the array. In some embodiments, reverse transcription can be performed to extend the 3' end of the capture probe using the captured analyte as a template. In some embodiments, a second strand hybridized to the extended capture probe can be generated (e.g., generated by any of the methods described herein), where the second strand includes a sequence complementary to the spatial barcode and at least a portion of the sequence of the analyte. Some embodiments of any of the methods described herein include releasing the second strand from the extended capture probe.

In some embodiments, the location of the analyte in the biological sample is determined by determining the sequence (using any of the exemplary sequencing methods described herein, e.g., high throughput sequencing) of the extended capture probe or the second strand.

Provided herein are methods for determining a location of an analyte in a biological sample that include adding an analyte capture sequence to the analyte in the biological sample, contacting the biological sample with an array comprising a plurality of capture probes, where a capture probe of the plurality of capture probes includes a spatial barcode and a capture domain that binds specifically to the analyte capture sequence, determining all or a part of the sequence of the spatial barcode, or a complement thereof, and all or a part of the portion of the sequence of the analyte that is specifically bound to the capture domain, or a complement thereof, and using the determined sequences of the spatial barcode, or complement thereof, and the analyte or complement thereof to identify the location of the analyte in the biological sample.

In some embodiments, determining all or a part of the sequence of the analyte that is specifically bound to the capture domain, or complement thereof, includes extending a 3' end of the capture probe using the analyte that is specifically bound to the capture domain as a template to generate an extended capture probe. In some embodiments, a second strand hybridized to the extended capture probe can be generated, where the second strand includes a sequence complementary to the spatial barcode and at least a portion of the sequence of the analyte. In some embodiments, the second strand can be used to identify the location of the analyte in the biological sample. In some embodiments, one or more labeled probes substantially complementary to the extended capture probe can be used to identify the location of the analyte in the biological sample. For example, one or more labeled (e.g., labeled with any detectable label described herein) probes can be provided to the array after generating the extended capture probe. The one or more labeled probes can specifically bind (e.g., hybridize) to the extended capture probe and can be detected, thus identifying the location of the analyte in the biological sample.

In some embodiments, a capture probe can further include a unique molecular identifier. In some embodiments, a capture probe can include one or more functional domains (e.g., one or more of any of the functional domains described herein). In some embodiments, a capture probe can include a cleavage domain. In some embodiments, the cleavage domain is positioned 5' to the capture domain in the capture probe. In some embodiments, a capture probe can include one or more of: a unique molecular identifier, one or more functional domains, and a cleavage domain positioned 5' to the capture domain.

In some embodiments, analytes can be migrated from the biological sample to the array including the plurality of capture probes. In some embodiments, the migration of analytes includes the use of passive migration (e.g., diffusion). In some embodiments, the migration of analytes includes the use of active migration using any of the exemplary methods described herein (e.g., electrophoresis). In some embodiments, the biological sample can be permeabilized (e.g., using any of the methods for permeabilizing a biological sample described herein) before adding an analyte capture sequence to analytes present in the biological sample. In some embodiments, the biological sample can be imaged (e.g., imaged by any of the methods described herein). In some embodiments, the biological sample is a tissue section (e.g., any of the exemplary tissue sections described herein) or any of the other types of biological samples described herein.

### RNA Integrity Number

As used herein, the term "RNA Integrity Number" or "RIN" refers to an indication of RNA quality based on an integrity score, the degree for which RNA from a sample is degraded or not (for example, as found in Schroeder, A., et al., The RIN: an RNA integrity number for assigning integrity values to RNA measurements, BMC Molecular Biology, 7:3 (2006) and Ahlfen, S.V., et al., Determinants of RNA Quality from FFPE Samples, PLoS ONE, 2(12): e1261 (2007), Mueller, O., et al, RNA Integrity Number (RIN) - Standardization of RNA Quality Control, Agilent Technologies (2004)). For example, a biological sample with a RIN score of about 1 includes RNA that is fully degraded, whereas a biological sample with a RIN score of about 10 includes RNA that is not degraded. In some embodiments, a RIN score can be calculated for a biological sample, one or more regions of a biological sample, or a single cell. In some embodiments, a RIN score can be calculated prior to performing any of the methods described herein on a similar biological sample. For example, a RIN score can be determined for a first biological sample and the second (e.g., next or adjacent) biological sample (e.g., tissue section) can be used to perform any of the methods described herein. In some examples, a RIN score of a first biological sample can be used to approximate the RIN value of the second (e.g., similar or adjacent) biological sample.

As used herein, the term "poor quality biological sample" refers to a biological sample with a RIN score of less than 5. In some embodiments, a poor quality biological sample can have a RIN score of less than 4.5, less than 4.0, less than 3.5, less than 3.0, less than 2.5, less than 2.0, less than 1.5, less than 1.0, less than 0.8, less than 0.6, less than 0.4, or less than 0.2. In some embodiments, the RIN value can be determined for a biological sample (e.g., a tissue section) before or after the biological sample is used for to perform any of the methods described herein. For example, a RIN value can be previously determined on a different (e.g., similar or adjacent) biological sample than the biological sample that is used to perform any of the methods described herein. In some embodiments, spatial analysis as described herein can be performed on samples with a RIN score between about 4 to about 10. In some embodiments, a poor quality biological sample can have a RIN score of between about 0.1 and about 5.0 (e.g., about 0.1 to about 4.5, about 0.1 to about 4.0, about 0.1 to about 3.5, about 0.1 to about 3.0, about 0.1 to about 2.5, about 0.1 to about 2.0, about 0.1 to about 1.5, about 0.1 to about 1.0, about 0.1 to about 0.8, about 0.1 to about 0.6, about 0.1 to about 0.4, about 0.1 to about 0.2, about 0.2 to about 5.0, about 0.2 to about 4.5, about 0.2 to about 4.0, about 0.2 to about 3.5, about 0.2 to about 3.0, about 0.2 to about 2.5, about 0.2 to about 2.0, about 0.2 to about 1.5, about 0.2 to about 1.0, about 0.2 to about 0.8, about 0.2 to about 0.6, about 0.2 to about 0.4, about 0.4 to about 5.0, about 0.4 to about 4.5, about 0.4 to about 4.0, about 0.4 to about 3.5, about 0.4 to about 3.0, about 0.4 to about 2.5, about 0.4 to about 2.0, about 0.4 to about 1.5, about 0.4 to about 1.0, about 0.4 to about 0.8, about 0.4 to about 0.6, about 0.6 to about 5.0, about 0.6 to about 4.5, about 0.6 to about 4.0, about 0.6 to about 3.5, about 0.6 to about 3.0, about 0.6 to about 2.5, about 0.6 to about 2.0, about 0.6 to about 1.5, about 0.6 to about 1.0, about 0.6 to about 0.8, about 0.8 to about 5.0, about 0.8 to about 4.5, about 0.8 to about 4.0, about 0.8 to about 3.5, about 0.8 to about 3.0, about 0.8 to about 2.5, about 0.8 to about 2.0, about 0.8 to about 1.5, about 0.8 to about 1.0, about 1.0 to about 5.0, about 1.0 to about 4.5, about 1.0 to about 4.0, about 1.0 to about 3.5, about 1.0 to about 3.0, about 1.0 to about 2.5, about 1.0 to about 2.0, about 1.0 to about 1.5, about 1.5 to about 5.0, about 1.5 to about 4.5, about 1.5 to about 4.0, about 1.5 to about 3.5, about 1.5 to about 3.0, about 1.5 to about 2.5, about 1.5 to about 2.0, about 2.0 to about 5.0, about 2.0 to about 4.5, about 2.0 to about 4.0, about 2.0 to about 3.5, about 2.0 to about 3.0, about 2.0 to about 2.5, about 2.5 to about 5.0, about 2.5 to about 4.5, about 2.5 to about 4.0, about 2.5 to about 3.5, about 2.5 to about 3.0, about 3.0 to about 5.0, about 3.0 to about 4.5, about 3.0 to about 4.0, about 3.0 to about 3.5, about 3.5 to about 5.0, about 3.5 to about 4.5, about 3.5 to about 4.0, about 4.0 to about 5.0, about 4.0 to about 4.5, or about 4.5 to about 5.0).

In some embodiments, one or more RIN scores for a given biological sample (e.g., tissue section, one or more regions of a tissue, or a single cell) are calculated by providing an array including a plurality of capture probes on a substrate, where a capture probe includes a capture domain and a spatial barcode, the biological sample can be stained with a histology stain (e.g., any of the stains described herein), contacting the array with the biological sample (e.g., tissue section), capturing an analyte (e.g., an 18S rRNA molecule) from the biological sample with the capture domain, generating a cDNA molecule (e.g., an extended capture probe) from the captured biological analyte (e.g., 18S rRNA), hybridizing one or more labeled oligonucleotide probes to the cDNA, imaging the labeled cDNA and the histology stain, and generating a RNA integrity number for a location in the spatial array, where the RNA integrity number includes an analysis of a labeled cDNA image and a histology stain (e.g., any of the stains described herein) image for the location.

In some embodiments, the biological sample (e.g., tissue section) is stained with a histology stain. As used herein, a "histology stain" can be any stain described herein. For example, the biological sample can be stained with any of the exemplary IF/IHC stains described herein. In some embodiments, the biological sample (e.g., tissue section) can be stained with hematoxylin and eosin. In some embodiments, the biological sample (e.g., tissue section) is stained with a histology stain (e.g., any of the stains described herein) before, contemporaneously with, or after labelling the cDNA with labeled oligonucleotide probes. In some embodiments, the stained biological sample can be, optionally, destained (e.g., using any of the exemplary methods described herein, e.g., washed in HCl). For example, hematoxylin, from an H&E stain, can be optionally removed from the biological sample by washing in dilute HCl (0.01 M) prior to further processing. In some embodiments, the stained biological sample can be optionally destained after imaging and prior to permeabilization.

In some embodiments, the array includes a plurality of capture probes on a substrate or on features on a substrate, where the capture probes include a capture domain and a spatial barcode. In some embodiments, the capture domain includes a homonucleotide sequence. In some embodiments, the homonucleotide sequence is a poly(T) sequence. For example, a capture domain can include a poly(T) sequence capable of capturing an 18S rRNA transcript, which had been previously tagged with a poly(A) sequence (e.g., homopolymeric or heteropolymeric sequence), from the biological sample.

In some embodiments, calculating one or more RIN scores for a biological sample can include hybridizing at least one (e.g., at least two, at least three, at least four, or at least five) labeled oligonucleotide probes to the cDNA (e.g., extended capture probe) generated from the 18S rRNA. In some embodiments, a labeled oligonucleotide probe includes a sequence that is substantially complementary to a portion of the 18S cDNA (e.g., extended capture probe). In some embodiments, four labeled oligonucleotide probes (P1-P4) are designed to hybridize at four different locations spanning the entire sequence of the 18S rRNA. In some embodiments, a labeled oligonucleotide probe can include any of the detectable labels as described herein. For example, an oligonucleotide labeled probe can include a fluorescent label (e.g., any of the fluorescent labels described herein, e.g., Cy3). In some embodiments, two or more of the labeled oligonucleotide probes designed with substantial complementarity to different locations within the 18S cDNA sequence include the same detectable label. For example, four labeled oligonucleotide probes, (P1-P4) each can be designed to have substantial complementarity to a different location within the 18S cDNA sequence and can all have the same detectable label (e.g., Cy3). In some embodiments, two or more of the labeled oligonucleotide probes designed with substantial complementarity to different locations within the 18S cDNA (e.g., extended capture probe) sequence can include a different detectable label. For example, four labeled oligonucleotide probes, (P1-P4) can each be designed to have substantial complementarity to a different location within the 18S cDNA (e.g., extended capture probe) sequence and can include different detectable labels.

In some embodiments, determining a RNA Integrity Number for a biological sample (e.g., tissue section, one or more regions of a tissue, or a single cell) includes analyzing the images taken from a spatial array and a histology stain (e.g., any of the stains described herein) for the same location. For example, for the array all images are generated by scanning with a laser (e.g., a 532-nm wavelength) after the fluorescently-labeled (e.g., Cy3-labeled) oligonucleotide probes have been hybridized to the 18S cDNA (e.g., extended capture probe). One image can be generated per probe (P1-P4) and one image can be generated where no fluorescently-labeled probes were hybridized (P0). Normalization of Fluorescence Units (FU) data is performed by subtraction of the auto-fluorescence recorded with P0 and division with P1. After alignment, the five images (one image from each probe, P1-P4, and one image from an area without bound probe) are loaded into a script. The script generates two different plots, one heat-map of RIN values and one image alignment error plot, which combines the histology stain (e.g., any of the stains described herein) image. The image alignment error plot is used to visualize which pixels and positions should be excluded from the analysis due to alignment errors between the images from P0-P4.

Sequence Listing
Poly(dT) Capture Domain
   SEQ ID NO: 1: TTTTTTTTTT
Custom sequencing primer
   SEQ ID NO: 2 AAGCAGTGGTATCAACGCAGAGTACATGGG
Template Switching Oligonucleotide with 10 bp randomer
   SEQ ID NO: 3 AAGCAGTGGTATCAACGCAGAGNNNNNNNNNN
x22_6mer
   SEQ ID NO: 4 TTGCTAGGACCGGCCTTAAAGCNNNNNN
x22_10mer
   SEQ ID NO: 5 TTGCTAGGACCGGCCTTAAAGCNNNNNNNNNN

### EXAMPLES

### Example 1. Processing FFPE Biological Samples

FFPE biological samples were initially incubated in a 40 °C water bath for attachment. Next, the FFPE biological samples were dried at 40 °C for 2 hours. Following the drying step, the FFPE biological samples were dewaxed and rehydrated with the following protocol: 2 x 10-minute washes in xylene; 2 x 3-minute washes in 100% ethanol, 1 x 3-minute wash in 96% ethanol, 1 x 3-minute wash in 70% ethanol, and 2 x 1-minute washes in H₂O. After dewaxing and rehydrating, the FFPE biological samples were H&E stained, dried, and imaged.

FFPE biological samples were pre-permeabilized with collagenase (0.2 U/µL) at 37 °C prior to decrosslinking formaldehyde-induced crosslinks. Next, to remove formaldehyde-induced crosslinks and/or modifications, FFPE biological samples were incubated in TE buffer (10 mM Tris, 1 mM EDTA, at pH 8.0) at 70 °C for 60 minutes. Following incubation in TE buffer, the FFPE biological samples were additionally permeabilized with pepsin for 30 minutes. The decrosslinked FFPE biological samples can then be contacted with an array followed by reverse transcription to extend the 3' end of a capture probe using a captured analyte as a template.

### Example 2. Spatial Analysis of Decrosslinked Mouse Brain FFPE Samples

Tables 1 and 1A below summarize the results of spatial analysis performed on mouse brain FFPE biological samples. The first two rows represent data obtained from fresh frozen tissue. Rows 3 and 4 represent data obtained from FFPE biological samples that were not treated with the decrosslinking protocol in Example 1 (control). Rows 5-7 represent data obtained from FFPE biological samples that were decrosslinked according to the protocol in Example 1. A significant increase in median genes per spot was observed between FFPE biological samples that were decrosslinked prior to spatial analysis as compared to FFPE biological samples that were not decrosslinked prior to spatial analysis. Also, a significant increase in median unique molecular identifier (UMI) counts per spot was observed between FFPE biological samples that were decrosslinked prior to spatial analysis as compared to FFPE biological samples that were not decrosslinked prior to spatial analysis.

**Table 1.**

| Analysis ID | Sample | Est. Number of Spots | Mean Reads per Spot | Reads Mapped Confidently to Transcriptome | Fraction Reads Usable | Number of Reads | Sequencing Saturation |
|---|---|---|---|---|---|---|---|
| 13382 | FF_rep1 | 1487 | 191833 | 83.9% | 40.4% | 285254943 | 66.5% |
| 133823 | FF_rep2 | 2027 | 151859 | 83.9% | 54.7% | 307819074 | 67.6% |
| 145249 | FFPE_E | 2763 | 16533 | 29.9% | 15.6% | 45679961 | 93.4% |
| 145250 | FFPE_G | 2616 | 18584 | 30.1% | 15.0% | 48615125 | 94.3% |
| 145843 | FFPE.TE.70C_D | 2659 | 23249 | 54.1% | 47.3% | 61817897 | 56.6% |
| 145844 | FFPE.TE.70C_E | 2746 | 20182 | 52.6% | 46.1% | 55419965 | 54.3% |
| 145845 | FFPE.TE.70C_G | 2686 | 20930 | 45.8% | 40.7% | 56218597 | 39.7% |

**Table 1A.**

| Analysis ID | Sample | Median Genes per spot | Total Genes Detected | Median UMI Counts Per Spot | mm10 Median genes per spot (20k raw reads per spot) | mm10 Median UMI counts per spot (20k raw reads per spot) | Fraction ribosomal protein UMI counts | Fraction mitochondrial UMI counts |
|---|---|---|---|---|---|---|---|---|
| 13382 | FF_rep1 | 5821 | 20140 | 24241 | 2538 | 5942 | 7.4% | 17.9% |
| 133823 | FF_rep2 | 6091 | 20661 | 26382 | 3140 | 7998 | 7.0% | 17.9% |
| 145249 | FFPE_E | 91 | 12437 | 153 | 0 | 0 | 0.8% | 36.9% |
| 145250 | FFPE_G | 88 | 12241 | 144 | 0 | 0 | 0.8% | 33.7% |
| 145843 | FFPE.TE.70C_D | 1909 | 17953 | 4639 | 1792 | 4286 | 2.1% | 31.6% |
| 145844 | FFPE.TE.70C_E | 1726 | 17843 | 4046 | 1720 | 4026 | 2.1% | 31.0% |
| 145845 | FFPE.TE.70C_G | 2220 | 18179 | 5136 | 2172 | 4992 | 2.0% | 27.0% |

Tables 2 and 2A below summarize the results of deep sequenced, decrosslinked mouse brain FFPE biological samples and fresh frozen mouse brain tissues. The first two rows represent data obtained from fresh frozen mouse brain samples. Rows 3-5 represent data obtained from decrosslinked mouse brain FFPE biological samples. These data show that the sequencing libraries in the mouse brain FFPE biological samples are not as complex as fresh frozen tissues. However, the FFPE biological samples performed at 30% of the fresh frozen samples.

**Table 2.**

| Analysis ID | Name | Mean Reads Per Cell | Reads Mapped Confidently to the Transcriptome | Fraction reads usable | mm10 Median Genes per spot | mm10 Total Genes Detected |
|---|---|---|---|---|---|---|
| 134946 | NoCoverslip_rep1 | 119956 | 83.9% | 78.2% | 6424 | 21117 |
| 134947 | NoCoverslip_rep2 | 122539 | 83.9% | 76.4% | 6372 | 21122 |
| 149262 | V19L01_108_D Meta Sample | 75330 | 54.0% | 46.8% | 2967 | 18753 |
| 149263 | V19L01_108_E Meta Sample | 67042 | 52.4% | 45.5% | 2750 | 18711 |
| 149264 | V19L01_108_G Meta Sample | 68921 | 45.7% | 40.1% | 3613 | 19094 |

**Table 2A.**

| Analysis ID | Name | mm10 Total Genes Detected | Mm10 Median UMI Counts Per Spot | mm10 Median genes per spot (50k raw reads per spot) | mm10 Median UMI counts per spot (50k raw reads per spot) | Fraction ribosomal protein UMI counts | Fraction mitochondrial UMI counts |
|---|---|---|---|---|---|---|---|
| 134946 | NoCoverslip_rep1 | 21117 | 29676 | 5288 | 19757 | 7.8% | 17.8% |
| 134947 | NoCoverslip_rep2 | 21122 | 28374 | 5206 | 18848 | 7.6% | 17.2% |
| 149262 | V19L01_108_D Meta Sample | 18753 | 8213 | 2555 | 6723 | 2.0% | 28.6% |
| 149263 | V19L01_108_E Meta Sample | 18711 | 7279 | 2456 | 6308 | 2.1% | 27.8% |
| 149264 | V19L01_108_G Meta Sample | 19094 | 10088 | 3194 | 8470 | 2.0% | 24.9% |

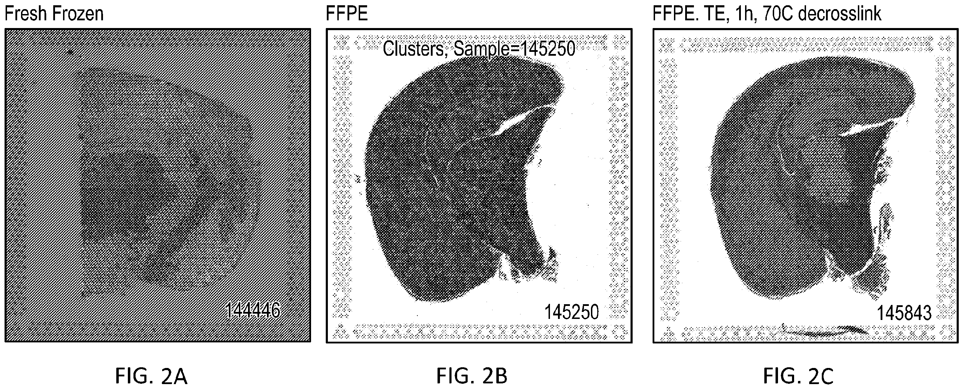

**FIGs. 2A-C** show spatial gene clustering in a fresh frozen sample and two different FFPE mouse brain samples. The data obtained using a fresh frozen biological sample is shown in **FIG. 2A****,** the data obtained using an FFPE biological sample without decrosslinking is shown in **FIG. 2B****,** and the data obtained using an FFPE biological sample with decrosslinking is shown in **FIG. 2C. FIG. 2C** shows spatial gene expression and clustering re-emerge (as compared to no clear clustering in **FIG. 2B**) similar to that of the fresh frozen sample (**FIG. 2A**) when the mouse brain FFPE biological sample was decrosslinked with TE buffer for 60 minutes at 70 °C.

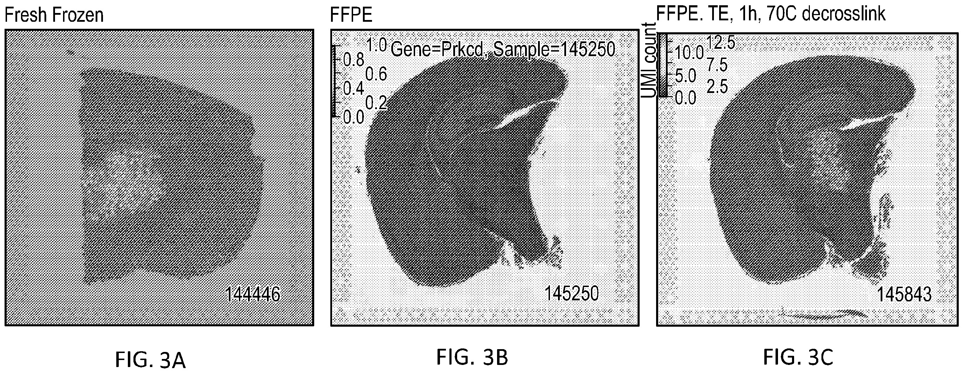

**FIGs. 3A-C** show spatial clustering of an individual gene (PRKCD) using mouse brain samples. The data obtained using a fresh frozen biological sample is shown in **FIG. 3A****,** the data obtained using an FFPE biological sample without decrosslinking is shown in **FIG. 3B****,** and the data obtained using an FFPE biological sample with decrosslinking is shown in **FIG. 3C. FIG. 3C** shows that spatial resolution of an individual gene (PRKCD) was observed similar to a fresh frozen sample (**FIG. 3A**) when the mouse brain FFPE biological sample was decrosslinked with TE buffer for 60 minutes at 70 °C.

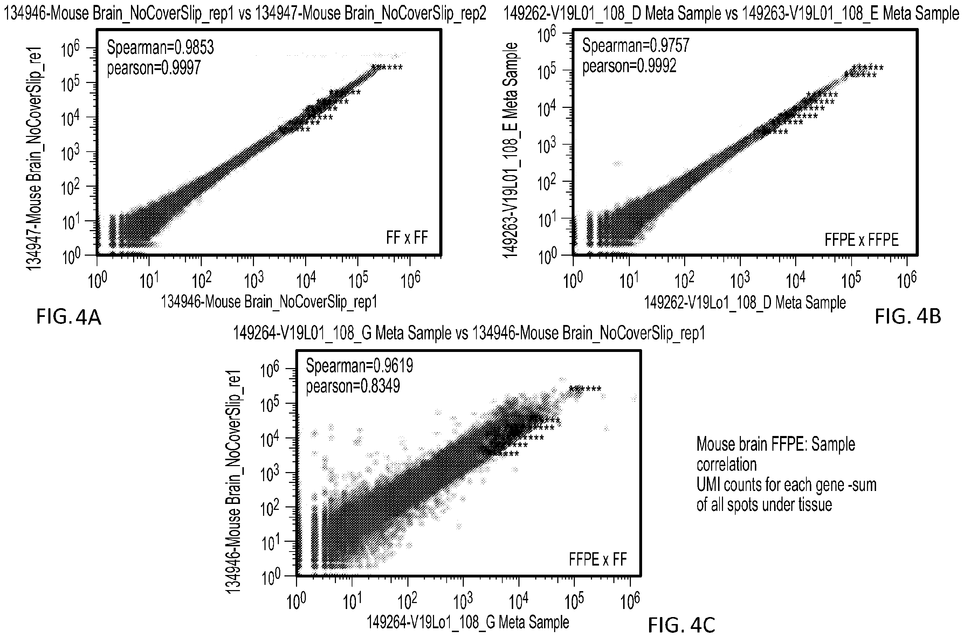

**FIGs. 4A-C** show sample correlation plots of data obtained from different biological samples. **FIG. 4A** is a correlation plot of data obtained from a fresh frozen sample and data obtained from a fresh frozen sample. **FIG. 4B** is a correlation plot of data obtained from a decrosslinked FFPE biological sample and data obtained from a decrosslinked FFPE biological sample. **FIG. 4C** is a correlation plot of data obtained from a decrosslinked FFPE biological sample and data obtained from a fresh frozen biological sample. These data demonstrate that the decrosslinking protocol for FFPE biological samples is highly reproducible.

### Example 3. Spatial Analysis on Decrosslinked Human Cancer FFPE Samples

Tables 3A, 3B, and 3C summarize spatial analysis performed on human cancer samples. Rows 1-4 represent the data obtained using fresh frozen samples from two individuals with invasive lobular carcinoma. Two triple-negative FFPE breast cancer samples were decrosslinked according to the protocol in Example 1. Rows 5-8 represent the data obtained using 7-year old metaplastic carcinoma FFPE biological samples. Rows 9-12 represent the data obtained using 4-year old ductal carcinoma biological samples. About 25-30% performance was achieved with 7-year old metaplastic carcinoma FFPE biological samples as compared to fresh frozen samples.

**Table 3A.**

| Analysis ID | Name | Mean Reads Per spot | Reads Mapped Confidently to the Transcriptome | Sequencing Saturation | Reads Mapped Confidently to Intronic Regions | Reads Mapped Confidently to Exonic Region |
|---|---|---|---|---|---|---|
| 149150 | FF_002_UserC_Huma nBreast InvasiveLobu | 56933 | 81.0% | 89.7% | 4.6% | 85.3% |
| 149151 | FF_002_UserC_Huma nBreast InvasiveLobu | 66504 | 75.8% | 89.1% | 4.8% | 79.4% |
| 149152 | FF_002_U_serD_Huma nBreast InvasiveLobu | 64191 | 84.7% | 83.7% | 3.2% | 89.0% |
| 149153 | FF_002_UserD_Huma nBreast InvasiveLobu | 104065 | 84.9% | 88.7% | 3.2% | 89.1% |
| 151683 | 7yearsold.71hfix_A | 25550 | 11.5% | 64.7% | 19.6% | 12.2% |
| 151684 | 7yearsold.71hfix_B | 21390 | 11.3% | 65.5% | 18.4% | 12.0% |
| 151685 | 7yearsold.71hfix_C | 21428 | 12.4% | 65.9% | 20.4% | 13.1% |
| 151686 | 7yearsold.71hfix_D | 23161 | 12.4% | 69.9% | 19.5% | 13.1% |
| 151687 | 4yearsold.24h fix_A | 16147 | 10.2% | 84.8% | 23.6% | 10.9% |
| 151688 | 4yearsold.24h fix_B | 15181 | 9.5% | 87.6% | 21.5% | 10.2% |
| 151689 | 4yearsold.24h fix_C | 13047 | 11.1% | 83.1% | 22.8% | 11.9% |
| 151690 | 4yearsold.24h fix_D | 14379 | 10.7% | 90.3% | 24.1% | 11.4% |

**Table 3B.**

| Analysis ID | Name | Fraction reads unmapped | Fraction reads with primer or homopolymer sequence | GRCh38 Median Genes per spot | GRch38 Total Genes Detected | GRCh38 Median UMI Counts per spot |
|---|---|---|---|---|---|---|
| 149150 | FF_002_UserC_Huma nBreast InvasiveLobu | 3.6% | 8.4% | 1432 | 21904 | 2485 |
| 149151 | FF_002_UserC_Huma nBreast_InvasiveLobu | 8.3% | 16.7% | 1562 | 21962 | 2676 |
| 149152 | FF_002_UserD_Huma nBreast InvasiveLobu | 2.6% | 5.8% | 1552 | 22575 | 3049 |
| 149153 | FF_002_UserD_Huma nBreast_InvasiveLobu | 2.6% | 5.8% | 2017 | 22986 | 4215 |
| 151683 | 7yearsold.71hfix_A | 49.7% | 32.6% | 458 | 17587 | 622 |
| 151684 | 7yearsold.71hfix_B | 50.7% | 36.0% | 351 | 17331 | 463 |
| 151685 | 7yearsold.71hfix_C | 48.8% | 31.5% | 413 | 17814 | 564 |
| 151686 | 7yearsold.71hfix_D | 49.5% | 32.2% | 398 | 17752 | 538 |
| 151687 | 4yearsold.24h fix_A | 50.8% | 35.5% | 138 | 16121 | 165 |
| 151688 | 4yearsold.24h fix_B | 51.9% | 40.2% | 86 | 14974 | 87 |
| 151689 | 4yearsold.24h fix_C | 50.6% | 32.9% | 140 | 16325 | 166 |
| 151690 | 4yearsold.24h fix_D | 50.5% | 32.5% | 64 | 15149 | 72 |

**Table 3C.**

| Analysis ID | Name | GRCh38 Median genes per cell (20k raw reads per spot) | GRCh38 Median genes per cell (5k raw reads per cell) | GRCh38 Median UMI counts per cell (20k raw reads per cell) | GRCh38 Median UMI counts per cell (5k raw reads per cell) | GRCh38 Fraction ribosomal protein UMI counts | GRCh38 Fraction mitochondri al UMI Counts |
|---|---|---|---|---|---|---|---|
| 149150 | FF_002_UserC_Huma nBreast InvasiveLobu | 1001 | 575 | 1588 | 813 | 24.1% | 2.1% |
| 149151 | FF_002_UserC_Huma nBreast InvasiveLobu | 1036 | 574 | 1608 | 796 | 22.3% | 1.9% |
| 149152 | FF_002_UserD_Huma nBreast InvasiveLobu | 1080 | 577 | 1968 | 888 | 28.7% | 2.2% |
| 149153 | FF_002_UserD_Huma nBreast InvasiveLobu | 1270 | 686 | 2382 | 1098 | 27.9% | 2.2% |
| 151683 | 7yearsold.71hfix_A | 420 | 200 | 565 | 253 | 4.4% | 3.2% |
| 151684 | 7yearsold.71hfix_B | 343 | 174 | 451 | 217 | 4.6% | 3.3% |
| 151685 | 7yearsold.71hfix_C | 404 | 211 | 550 | 267 | 4.2% | 4.7% |
| 151686 | 7yearsold.71hfix_D | 381 | 205 | 514 | 259 | 4.3% | 4.6% |
| 151687 | 4yearsold.24h fix_A | 0 | 102 | 0 | 118 | 3.9% | 2.6% |
| 151688 | 4yearsold.24h fix_B | 0 | 58 | 0 | 66 | 4.2% | 2.5% |
| 151689 | 4yearsold.24h fix_C | 0 | 109 | 0 | 127 | 4.1% | 3.7% |
| 151690 | 4yearsold.24h fix_D | 0 | 53 | 0 | 59 | 4.0% | 2.9% |

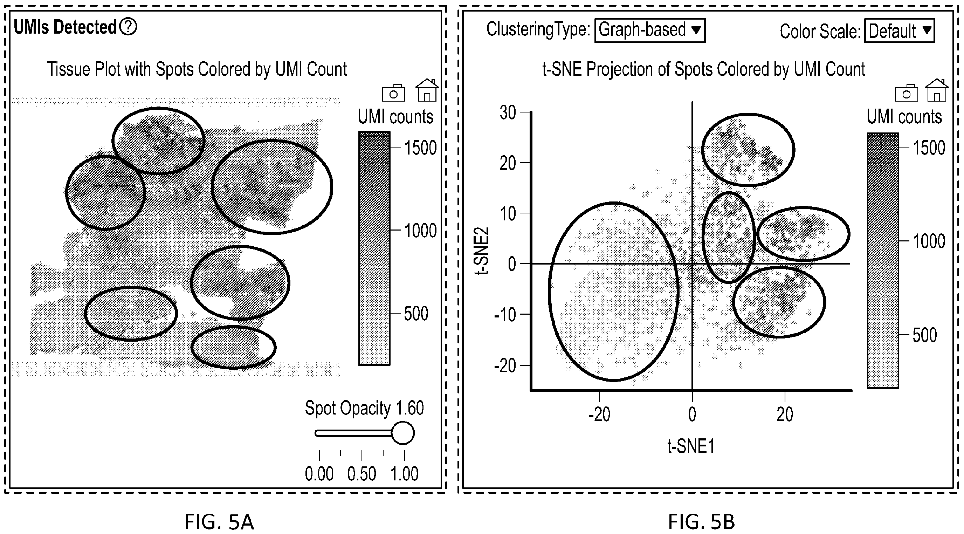

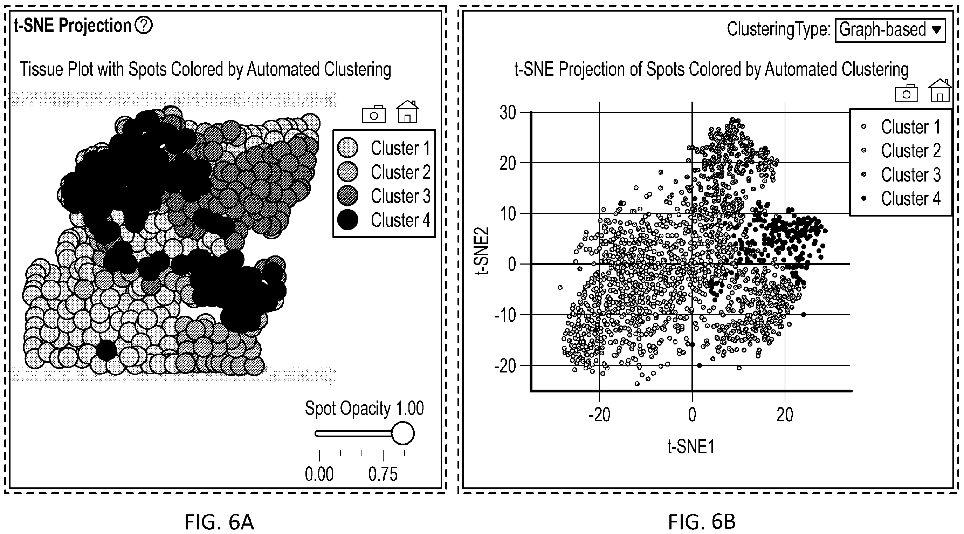

The data represented in **FIGs. 5A-B** and **FIGs. 6A-B** were generated from a decrosslinked 7-year old metaplastic carcinoma FFPE biological sample (sample 151683, row 5 in Tables 3A-C). **FIGs. 5A-B** show successful spatial clustering using a decrosslinked 7-year old metaplastic carcinoma FFPE biological sample. **FIG. 5A** is a tissue plot with spots colored by UMI count. **FIG. 5B** is a t-SNE projection of spots colored by UMI counts. **FIGs. 6A-B** also show successful spatial clustering from a decrosslinked 7-year old metaplastic carcinoma FFPE biological sample. **FIG. 6A** is a representation of a tissue plot with spots colored by automated clustering. **FIG. 6B** is a representation of a t-SNE projection of spots colored by automated clustering.

### Example 4. TdT/T4 DNA Ligase Splinted Ligation on Decrosslinked FFPE Samples

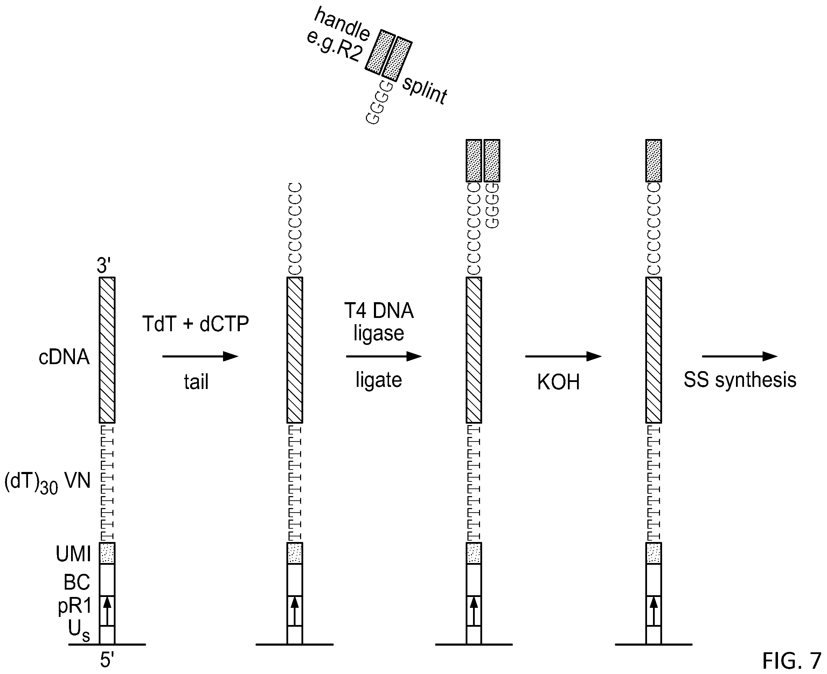

An exemplary method for improving library preparation efficiency in decrosslinked FFPE biological samples is shown in **FIG. 7****.** A protocol following the schematic can be used to add a poly(C) tail to the 3' end of cDNA generated by reverse transcription with a terminal deoxynucleotidyl transferase in the presence of dCTP. A partially double-stranded splint oligonucleotide is provided that contains a first oligonucleotide with a sequencing handle and a second oligonucleotide with a sequence in the 5' to 3' direction that includes a poly(G) sequence substantially complementary to the added poly(C) sequence and a single-stranded portion substantially complementary to the sequencing handle. The first oligonucleotide of the splint oligonucleotide is ligated to the poly(C) sequence with T4 DNA ligase. The second oligonucleotide of the splint oligonucleotide is dissociated from the capture probe containing the first oligonucleotide in the presence of KOH and second strand synthesis is performed with a primer substantially complementary to the sequencing handle ligated to the extended capture probe.

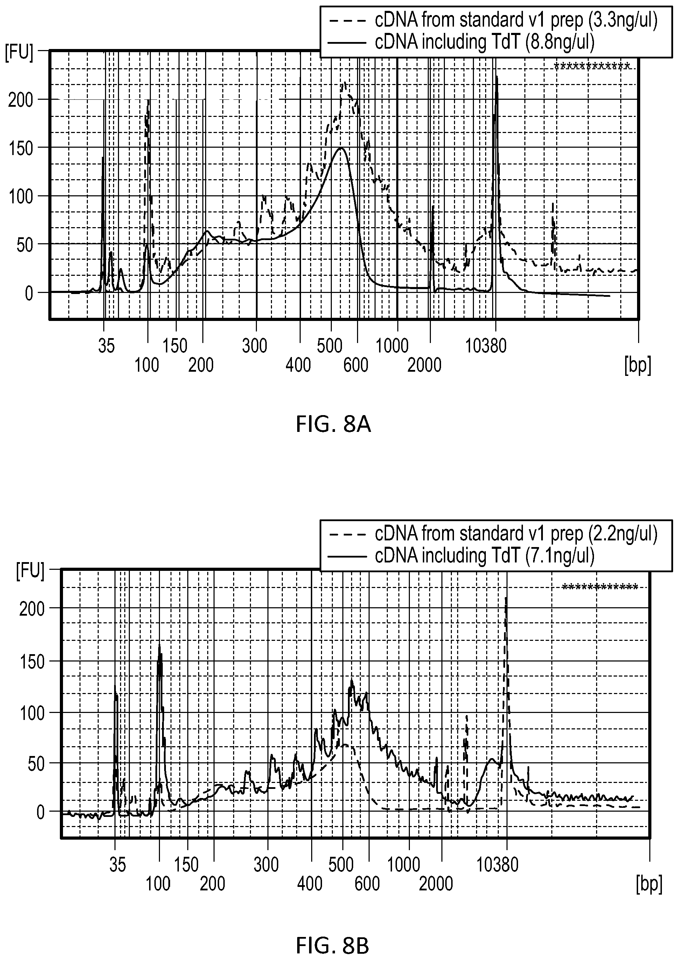

**FIGs. 8A-B** shows cDNA prepared from decrosslinked FFPE biological samples. For example, **FIG. 8A** shows cDNA generated from standard preparation (3.3 ng/µL) as compared with cDNA generated with terminal deoxynucleotidyl transferase (TdT) and T4 DNA ligase (8.8 ng/µL). **FIG. 8B** shows cDNA generated from standard preparation (2.2 ng/µL) as compared with cDNA generated with TdT and T4 DNA ligase (7.1 ng/µL). Both figures show that a higher yield was achieved with the TdT and ligation strategy.

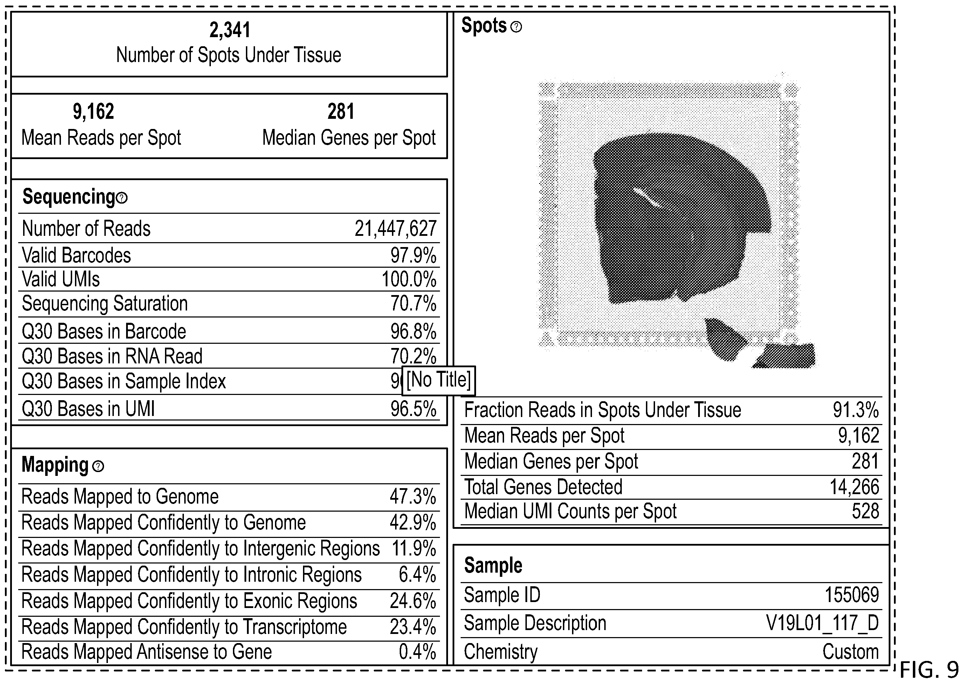

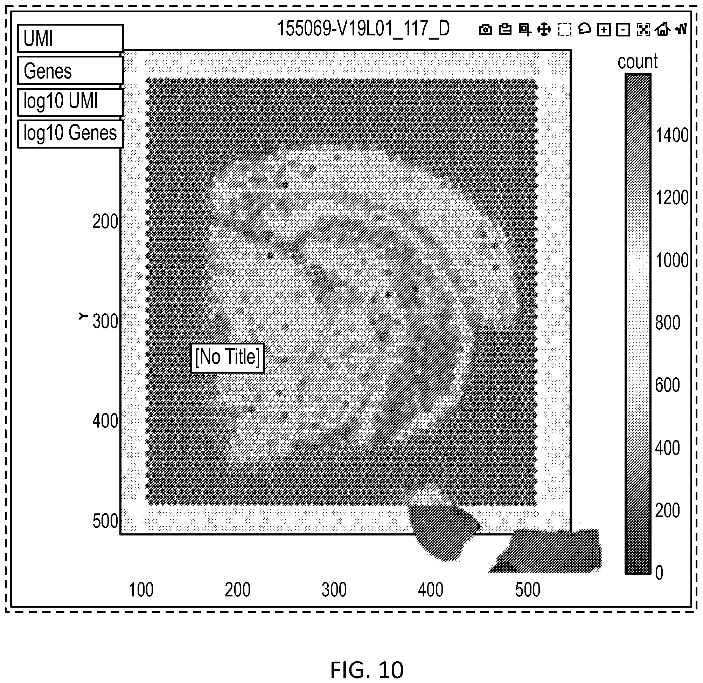

**FIGs. 9-10** show successful spatial analysis with decrosslinked FFPE biological samples where a TdT and T4 DNA ligase strategy was used to generate a second strand. **FIG. 10** shows successful spatial clustering in a decrosslinked FFPE biological sample that included a TdT and T4 DNA ligase strategy to ligate a sequencing handle to extended capture probes.

### Example 5. Second-Strand Synthesis with Random Primers on Decrosslinked FFPE Biological Samples

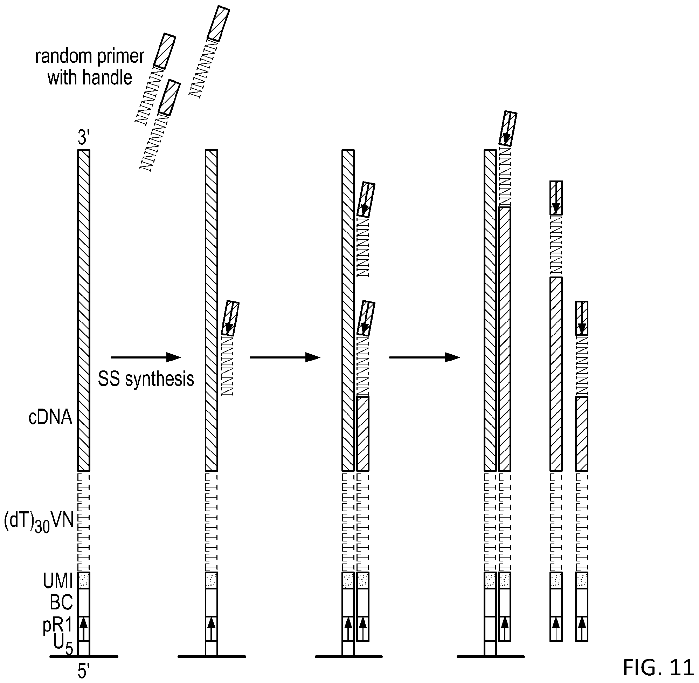

**FIG. 11** is a schematic diagram of a method that can be used to generate a second strand after reverse transcription using random primers. Random hexamer or octamer primers including a sequencing handle are provided to the array after the decrosslinked FFPE biological sample is removed from the array. The random primers hybridize to the extended capture probe at random locations after reverse transcription. More than one random primer can hybridize to an extended capture probe. A stand-displacing polymerase (Klenow fragment) is provided to extend the hybridized random primers and generate second strands. The reaction is performed at 37 °C.

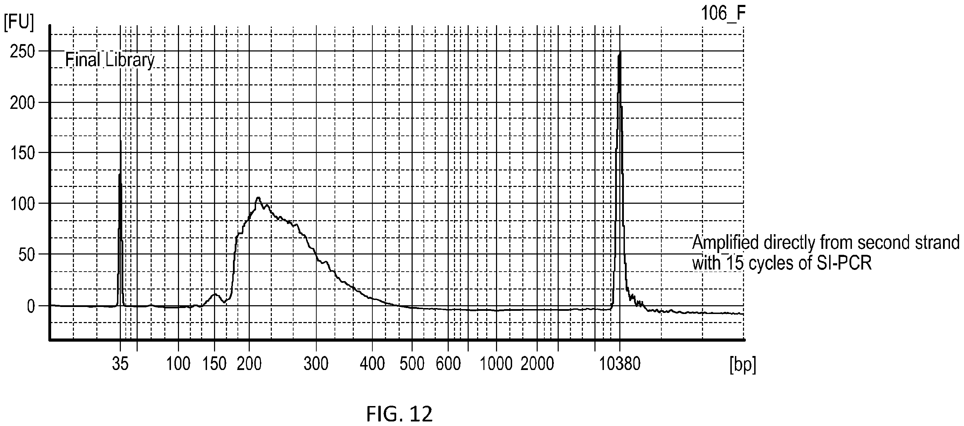

**FIG. 12** shows a sequencing library prepared using a decrosslinked FFPE biological sample (mouse brain) that had been fixed for 21 hours. The sequencing library was amplified directly from the second strand with 15 cycles of standard index-PCR. The final library was shorter than a library prepared from a fresh frozen sample, which was anticipated.

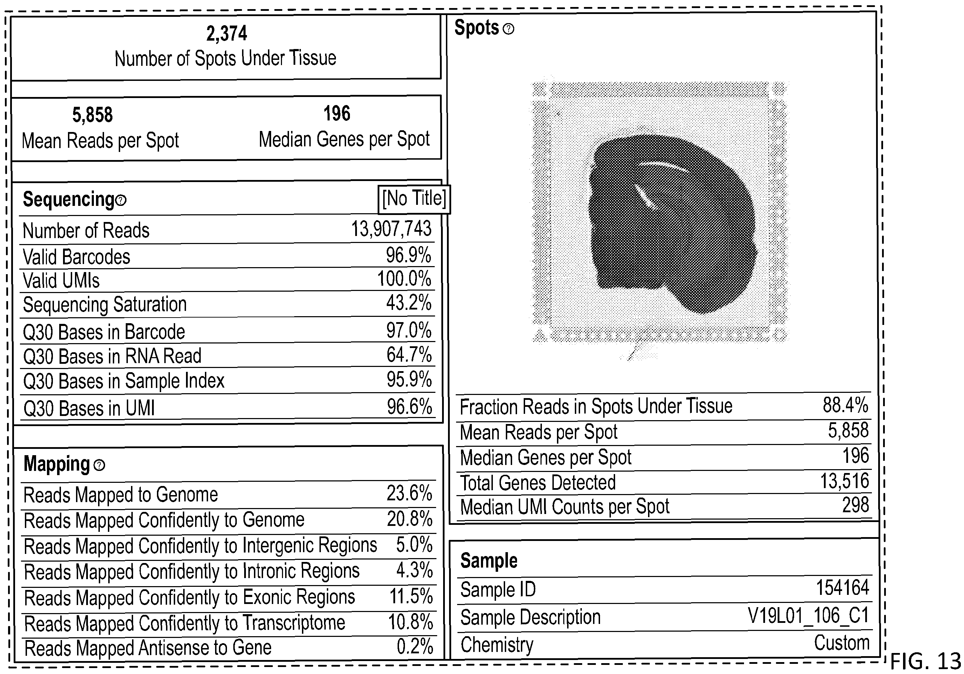

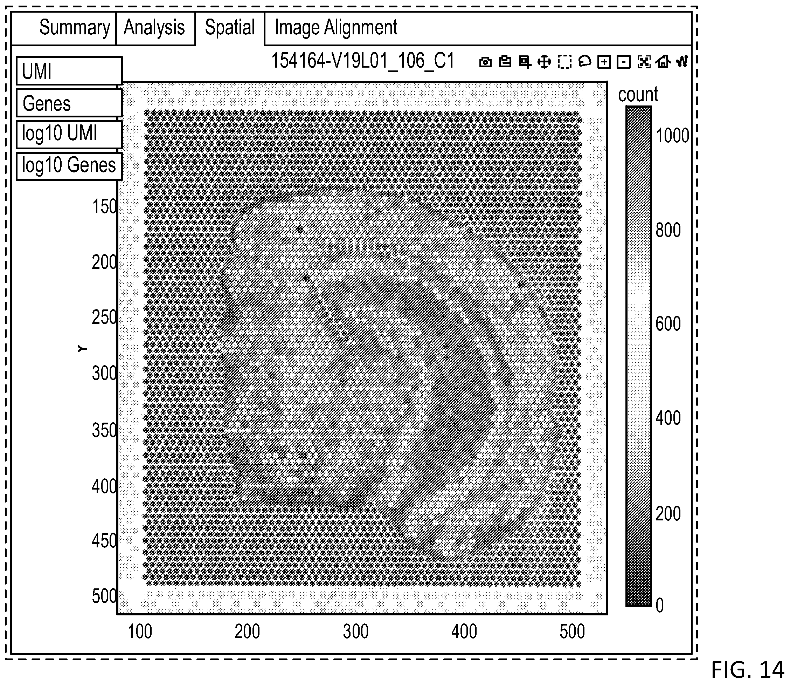

**FIGs. 13** and **14** show successful spatial analysis performed on decrosslinked FFPE biological samples where the second strands were generated with random primers including sequencing handles and a strand displacing polymerase.

### Example 6. Single-Stranded Ligation with 5' App DNA/RNA Ligase

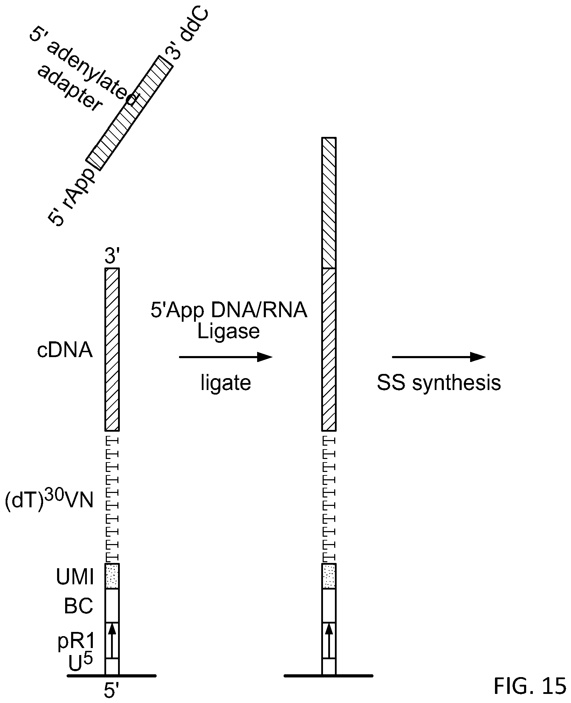

**FIG. 15** is a schematic diagram that can be used to ligate a sequencing handle to the 3' end of the extended capture probe after reverse transcription. In this example, a 5' adenylated adaptor can be provided to the decrosslinked FFPE biological sample after reverse transcription. The 5' adenylated adaptor can be ligated to the 3' end of the extended capture probe. The 3' end of the adenylated adaptor can also be blocked to prevent the 5' adenylated adaptor from ligating to itself. For example, the 3' end of the adenylated adaptor can have a 3'-terminal blocking group, such as a dideoxy nucleotide or a 3'-amino linker, which lack 3'-OH groups. The 5' adenylated adaptor can be ligated by a 5'App DNA/RNA ligase. A primer substantially complementary to the sequencing handle in the ligated 5' adenylated adaptor can hybridize to the sequencing handle and be extended to generate a second strand.

### Example 7. Single-Stranded Ligation using TACS Ligation

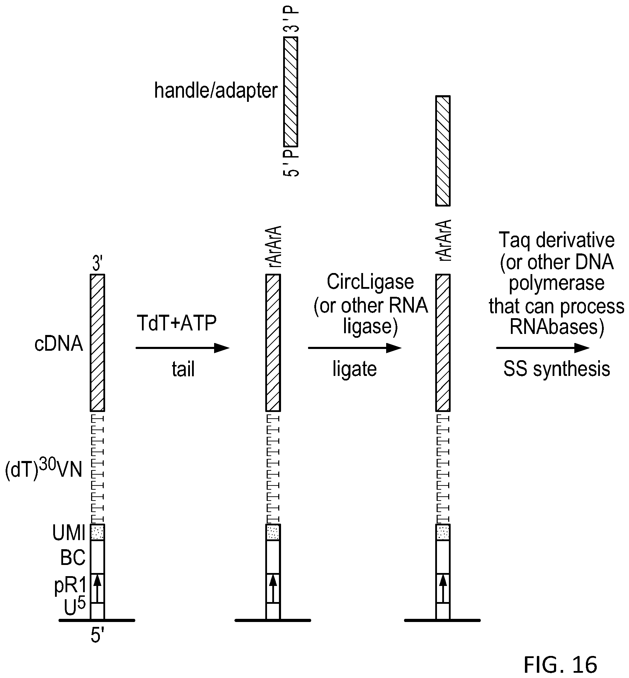

**FIG. 16** is a schematic diagram of a method that can be used to ligate a sequencing handle to the 3' end of the extended capture probe after reverse transcription. In this example, a poly(A) sequence can be added to the 3' end of the extended capture probe. The poly(A) sequence can be a ribonucleic acid sequence and can be added with TdT in the presence of ATP. An adaptor including a sequencing handle can be ligated to the ribonucleic poly(A) sequence with an RNA ligase. The RNA ligase can be CircLigase. A primer substantially complementary to the sequencing handle in the adaptor can hybridize to the sequencing handle and be extended to generate a second strand. The primer can be extended with a DNA polymerase that can process RNA bases as a substrate. The DNA polymerase can be a Taq species derivatized polymerase.

### Example 8. Processing FFPE Biological Samples

A FFPE Mouse Brain sample from a male mouse weighing 25 g, and being 8-12 weeks of age (sample number C57BL6J (Adlego Biomedical)) was extracted under ethical permit number 4570-2019. The RIN was calculated to be 2.9 and the DV200 was at 65%.

A FFPE Gynecological Carcinosarcoma sample of high grade serous ovarian carcinosarcoma metastasis to the omentum was extracted under ethical permit number 2018/2264-31. The RIN and DV200 for two regions was calculated: 1919-1 sample RIN 2.5, DV200 69% and 1919-2 sample RIN 2.30, DV200 64%.

Sectioning, deparaffinization and staining of FFPE samples: Gynecological carcinosarcoma microtome sections (12 µm thick) were placed on spatial array slides (Visium, 10X Genomics) after floating on a water bath at 43°C. The process was repeated for FFPE mouse brain (10 µm thick) and an FFPE lung SARS-CoV-2 infected biopsy (10 µm thick). After sectioning, the slides were dried at 40°C in an oven for 1 hour and 45 minutes. The slides were then placed inside a slide mailer, sealed with parafilm and left overnight at 4°C. The slides were deparaffinized by immersion in the following reagents and the specified durations: xylene 7 min, xylene 7 min, EtOH 99% 2 min, EtOH 99% 2 min, EtOH 96% 2 min, EtOH 96% 2 min. Staining was performed according to the Visium Spatial Gene Expression Reagent Kits User Guide (10x Genomics, Inc, https://support.10xgenomics.com/spatial-gene-expression/library-prep/doc/user-guide-visium-spatial-gene-expression-reagent-kits-user-guide) Step 1.3, with the following modifications: Step 1.3.q slides were incubated in Dako bluing buffer (#CS70230-2 Agilent) for 30 seconds. After Step 1.3.z slides were mounted with 200 µl 85% glycerol 85% and a cover glass was applied to the slide.

H&E imaging: Slides were scanned under a high-resolution microscope to obtain tissue images. After imaging, the glycerol and coverslips were removed by holding the slides in an 800 ml beaker and letting the glycerol diffuse until the coverslip was displaced and density changes were no longer visible in the water. The slides were then dried at 37°C.

Analyte retrieval and Permeabilization: The slides were mounted in an Array-It metallic incubation chamber. Collagenase was equilibrated to 37°C and 75 µl was added to each well. The slides were sealed and incubated for 20 min at 37°C in a Thermo-block with a heated lid. After incubation the collagenase was pipetted off and the wells were rinsed by adding and removing 100 µl of 0.1 x SSC buffer to each well. Next 100 µl of Tris-EDTA (TE) buffer was added to each well and the slides were sealed and incubated for 1 hour at 70°C in a Thermo-block with a heated lid. After incubation, the slides were removed and equilibrated at room temperature for 5 min. Next, 0.1% pepsin solution (P7000-25G) was dissolved in 0.1M HCl (#319865-1000ML Sigma Aldrich) and equilibrated at 37°C. After incubation and one wash per well with 100 µl 0.1 x SSC buffer, the tissue sample was permeabilized with 75 µl of the prepared pepsin solution and the slides were sealed and incubated with a heated lid at 37°C for 30 minutes. Reverse transcription was performed as described in step 1.2 of the User Guide, with the exception that the slides were masked in Array-It metallic incubation cassettes instead of Visium Slide cassettes.

Second strand synthesis and denaturation: second strand synthesis and denaturation were performed according to the standard User Guide (step 2), however, the slides were masked in Array-It metallic incubation chambers rather than slide cassettes.

cDNA amplification, Cleanup and Quantification: cDNA obtained from mouse brain and sarcomas were amplified by 14 cycles of PCR with settings according to the User Guide (step 3.2). The samples were purified with 0.8x SPRIselect bead clean-up, rather than the specified 0.6x in the Visium Spatial Gene Expression, thus 80 µl of SPRIselect beads were added to 100 µl of sample instead of 60 µl (step 3.3.a). Additionally, elution buffer volume was reduced to 15 µl rather than 40.5 µl (step 3.3.j). cDNA yield was checked using 1 µl of sample for quality control on an Agilent BioAnalyzer High Sensitivity chip (step 3.4).

Fragmentation, end repair and A-tailing: 10 µl of each sample was fragmented in a thermal cycler (step 4.1) with a fragmentation run time of 1 min instead of 5 min. The samples were cleaned up post-fragmentation by a 0.8x SPRI bead selection step. 40 µl of SPRIselect beads were added to 50 µl of sample and incubated at room temperature for 5 min. The tubes were placed on the high magnet position until the solution cleared. The supernatant was discarded and the beads were washed twice for 30 seconds with 125 µl of 80% ethanol. After removing the 80% ethanol, beads were allowed to dry on the magnet for about 2 min (avoiding over-drying) and then 50.5 µl of elution buffer was added to each sample. The samples were removed from the magnet, vortexed and spun down briefly, then incubated for 2 min at room temperature. After incubation the samples were placed on the low magnet position until the solution cleared. 50 µl of each sample was transferred to a new tube strip.

Adapter Ligation SPRIselect post-ligation clean-up: These steps were performed as according to the instructions described in the User Guide (step 4.3 and 4.4).

Sample Index PCR and clean-up: The samples were indexed according to the manufacturer's instructions in the User Guide (step 4.5), with 8 cycles of PCR. The samples were cleaned up according to step 4.6, however, 40.5 µl of elution buffer (EB) were added instead of 35.5 µl resulting in a final eluted volume of 40 µl which was transferred to a new tube strip. An additional 0.8x SPRI select bead purification step was also performed (32 µl of SPRIselect beads were added to 40 µl of sample and incubated at room temperature for 5 min). The tubes were placed on the high magnet position until the solution cleared. The supernatant was discarded and the beads were rinsed twice for 30 seconds in 200 µl of 80% ethanol. After rinsing, the beads were allowed to dry on the magnet for about 2 min (avoiding over-drying) and 15 µl of elution buffer was added to each sample. The samples were removed from the magnet, vortexed, and centrifuged briefly, and then incubated for 2 min at room temperature. After incubation the samples were placed on the low magnet position until the solution cleared. The eluted samples were transferred to a new tube strip.

Post-library Quality Control and dilution: Post-library quality control and dilution was performed according to the User Guide (step 4.7). In addition, 2 µl of each sample were used for dsDNA high sensitivity Qubit Assay (ThermoFisher Scientific) to determine sample concentration.

Sequencing: Libraries were sequenced using Illumina's Nextseq 500. A total of 4 samples per run, with 75 cycles High Output kits, with paired-end, dual index sequencing and a custom sequencing primer (SEQ ID NO: 2) instead of the Read 2 primer. A total of 2 ml of 0.3 µM of the custom sequencing primer were loaded into well number 8 of the sequencing cartridge. The loading concentration of the libraries was 1.8 pM with a 1% PhiX spike-in. Read 1: 28 cycles, i7 index: 10 cycles, i5 index: 10 cycles, custom sequencing primer (SEQ ID NO: 2): 44 cycles (instead of 120 cycles in the manufacturer's instructions).

Data pre-processing: The raw fastq files containing the cDNA sequences (Read 2) were pre-processed to remove template switching oligonucleotide (TSO) primer sequences and poly(A) homopolymers using cutadapt (v2.8) 3. TSO sequences were trimmed by defining the TSO sequence as a non-internal 5' adapter (which will remove partial or full TSO sequences from the 5' end) with a minimum overlap of 5 bp and an error tolerance of 0.1. Poly(A) homopolymers were trimmed by defining 10 A's as a regular 3' adapter (which will remove stretches of poly(A) found anywhere in the sequence as well as the trailing base pairs with a minimum overlap of 5bp). To search for and trim both adapter types from the same read sequences the times option was set to 2.

Data Processing: All paired fastq files (after TSO and poly(A) trimming) were processed using Space Ranger v1.0.0 together with the corresponding Hematoxylin and Eosin (H&E) stained images in jpeg format. For mapping of the data, mm 10-3.0.0 *mus musculus* reference genome was used for mouse samples and the GRCh38-3.0.0 homo sapiens reference genome for human samples (both included in the Space Ranger distribution v1.0.0).

### Example 9. Processing PFA-fixed Biological Samples

Mouse brain samples were fixed on-slide at 1%, 2%, and 4% with paraformaldehyde (PFA) for 10 minutes followed by cold methanol incubation for 5 minutes. Standard H&E staining followed the cold methanol incubation. The mouse brain samples were decrosslinked with 20 mM Tris-HCl at pH 9.0 at 60°C for 1 hour. Following de-crosslinking, labeled cDNA was generated from captured nucleic acids on the array. Mouse brain samples were washed in 1x PBS (100 µl) two times. Various permeabilization times with pepsin were tested including: no pepsin, 10 minutes, 20 minutes, 30 minutes, 40 minutes, 50 minutes, 60 minutes, and 70 minutes.

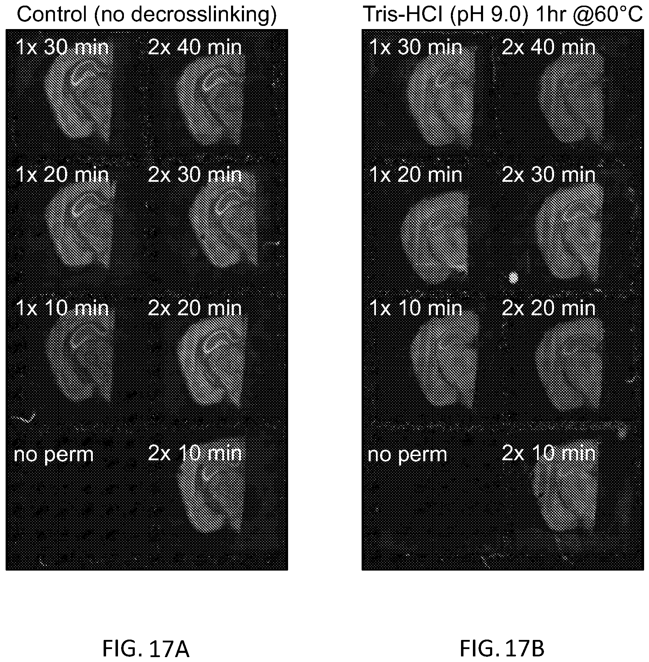

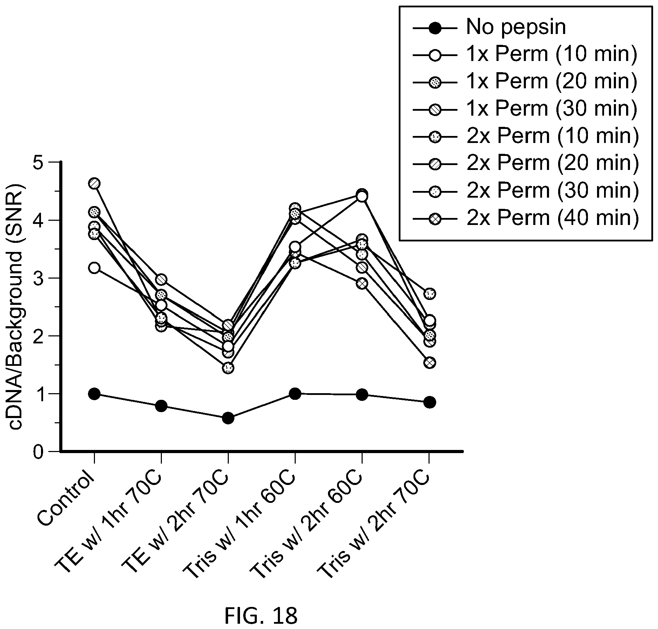

**FIG. 17A** shows labeled cDNA in control mouse brain samples where no decrosslinking had been performed under various permeabilization conditions (e.g., number of permeabilization incubations and duration) as indicated in **FIG. 17A. FIG. 17B** shows labeled cDNA in mouse brain samples where the samples (1% PFA fixed) were decrosslinked with Tris-HCl (pH 9.0) at 60°C for 1 hour and permeabilized under various conditions (e.g., number of permeabilization incubations and duration) as indicated in **FIG. 17B****.** **FIG. 18** is a graph showing the signal to noise ratio of the labeled cDNA to background noise (y-axis) under various permeabilization times and decrosslinking conditions (x-axis). The bottom line in **FIG. 18** represents a control sample with no permeabilization (e.g., no pepsin) and little to no signal was detected over the background noise. The data represent improved fluorescent signal in 20 mM Tris-HCl treated samples, such as the 20 mM Tris-HCl treated samples with 1 or 2 decrosslinking periods and with 10 minute permeabilization incubation(s). Additionally, increasing the decrosslinking duration appeared to reduce the signal to noise ratio within TE buffer treated samples.

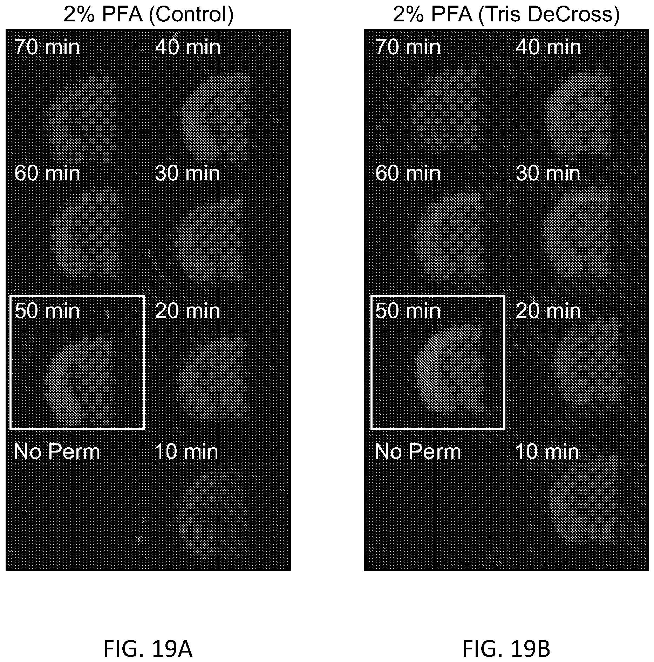

**FIGs. 19A** and **19B** show cDNA signal detected in either a control set of samples **(****FIG. 19A****)** with no decrosslinking performed under varying permeabilization durations as indicated in **FIG. 19A** or a set of decrosslinked samples **(****FIG. 19B****)** under varying permeabilization durations as indicated in **FIG. 19B****.** Both non-decrosslinked and decrosslinked samples shown in **FIGs. 19A-B** were previously fixed with 2% PFA. The images show that for this experiment a 50 minute permeabilization incubation provided the strongest signal after decrosslinking **(****FIG. 19B****).**

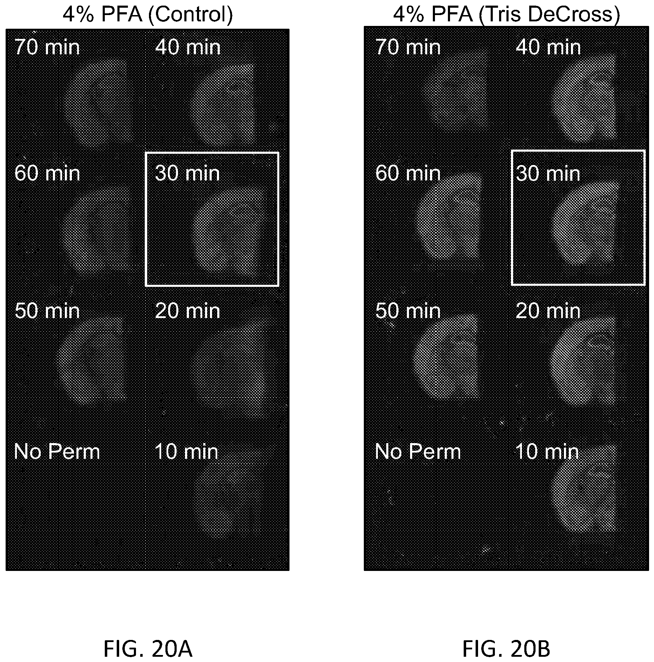

**FIGs. 20A** and **20B** show cDNA signal detected in either a control set of samples **(****FIG. 20A****)** with no decrosslinking performed under varying permeabilization durations as indicated in **FIG. 20A** or a set of decrosslinked samples **(****FIG. 20B****)** under varying permeabilization durations as indicated in **FIG. 20B****.** Both non-decrosslinked and decrosslinked samples shown in **FIGs. 20A** and **20B** were previously fixed with 4% PFA. The images show that for this experiment a 30 minute permeabilization provided the strongest signal after decrosslinking **(****FIG. 20B****).**

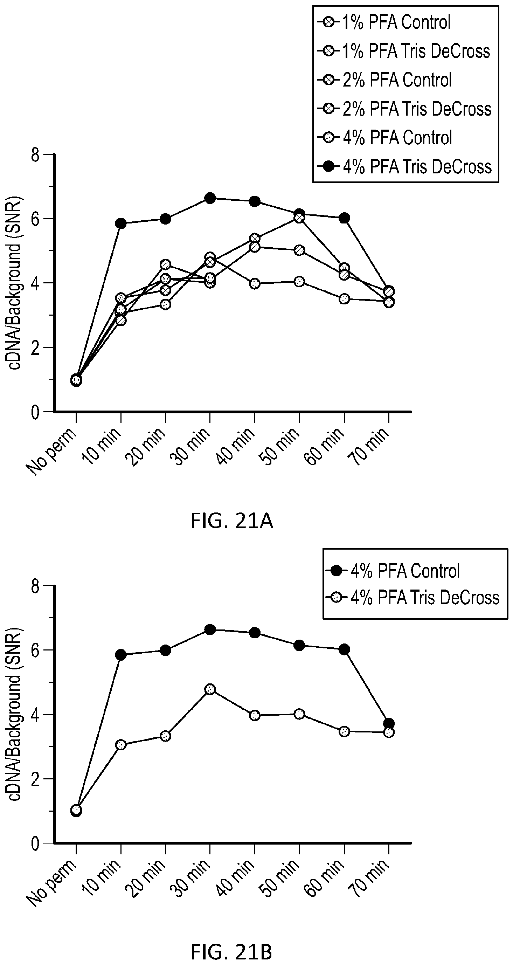

**FIGs. 21A** and **21B** are graphs showing the signal to noise ratio of labeled cDNA to background (y-axis) of samples under different PFA fixation conditions (e.g., 1%, 2%, or 4% PFA) and different permeabilization durations (e.g., 10, 20, 30, 40, 50, 60, or 70 minutes) (x-axis). **FIG. 21B** shows the 4% PFA fixed control and the decrosslinked 4% PFA fixed sample from **FIG. 21A****.** The data show that decrosslinking improves signal detection from on-slide PFA fixed samples. The data also indicate that increased permeabilization durations do not necessarily result in an increased cDNA/background noise ratio.

### Example 10. Processing Cardiac Perfused PFA-fixed Biological Samples (Cardiac Perfused Data)

Mouse cardiac tissue was perfused prior to tissue isolation with 1x PBS followed by 4% PFA solution. The perfused samples were then incubated in cold methanol for 5 minutes. Standard H&E staining followed the cold methanol incubation. The mouse brain samples were decrosslinked with 20 mM Tris-HCl at pH 9.0 at 60°C for 1 hour. Following decrosslinking, labeled cDNA was generated from captured nucleic acid on the array. Mouse brain samples were washed in 1x PBS (100 µl) two times. Various permeabilization times with pepsin were tested including: no pepsin (control), 10 minutes, 20 minutes, 30 minutes, 40 minutes, 50 minutes, 60 minutes, or 70 minutes.

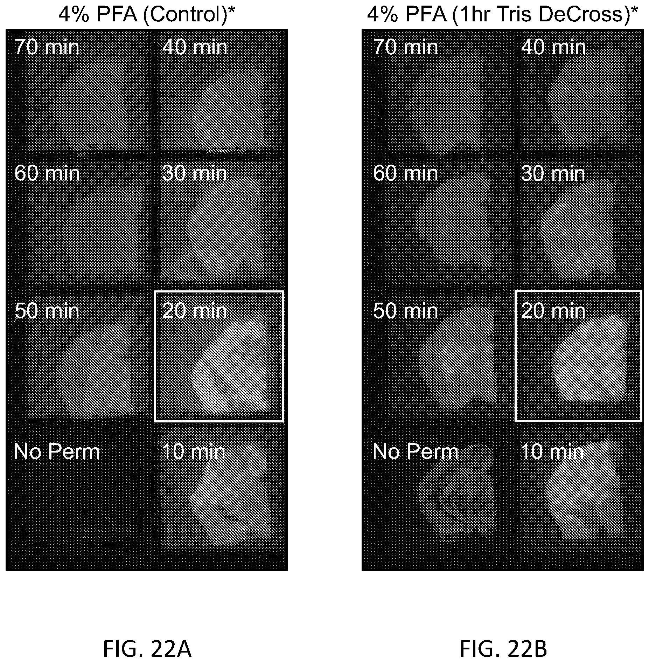

**FIGs. 22A** and **22B** show labeled cDNA signal detected in either a control set of samples **(****FIG. 22A****)** with no decrosslinking performed under varying permeabilization durations as indicated in **FIG. 22A** or a set of decrosslinked samples **(****FIG. 22B****)** under varying permeabilization durations as indicated in **FIG. 22B****.** Both non-decrosslinked and decrosslinked samples shown in **FIGs. 22A** and **22B** were previously fixed with 4% PFA. The images show that for this experiment a 20 minute permeabilization incubation provided the strongest signal after decrosslinking **(****FIG. 22B****).**

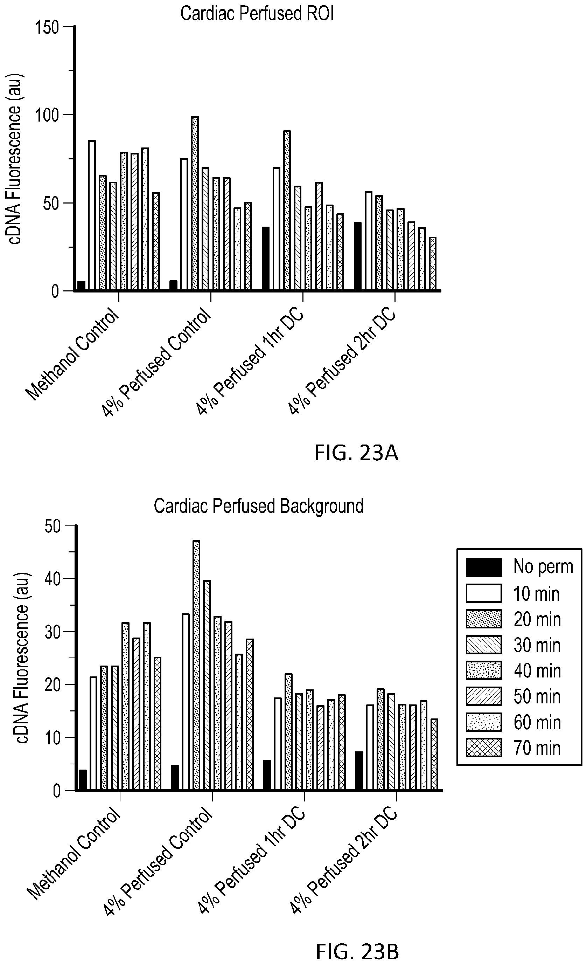

**FIGs. 23A** and **23B** are graphs showing total cDNA fluorescence (y-axis) detection from a methanol control sample, a 4% PFA fixed control sample, and a 4% PFA fixed sample that was de-crosslinked for either 1 hour or 2 hours from the biological sample **(****FIG. 23A****)** or background fluorescence **(****FIG. 23B****).** The samples were permeabilized for 10, 20, 30, 40, 50, 60, or 70 minutes, or not permeabilized (e.g., no pepsin) at all (control sample).

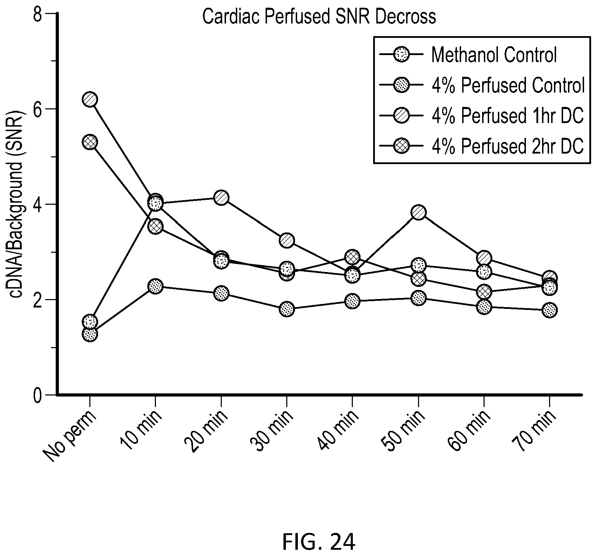

**FIG. 24** is a graph showing the signal to noise ratio of the labeled cDNA to background fluorescence (y-axis) under various permeabilization times (x-axis) and decrosslinking conditions. The data show that the 4% cardiac perfused samples generated substantial background signal **(****FIG. 23A****).** Cardiac perfused samples that were decrosslinked as described above resulted in increased signal to noise ratios and a prolonged decrosslinking incubation (e.g., 2 hours) resulted in a decrease in fluorescent signal.

### Example 11. Gene Expression Analysis in PFA-fixed Biological Samples

Mouse brain samples were fixed on-slide in either 1%, 2%, or 4% PFA for 10 minutes followed by a 5 minute cold methanol incubation. Standard H&E staining followed the cold methanol incubation. The mouse brain samples were decrosslinked with 20 mM Tris-HCl at pH 9.0 at 60°C for 1 hour. Following de-crosslinking, labeled cDNA was generated from captured nucleic acid on the array. Mouse brain samples were washed in 1x PBS (100 µl) two times. Various permeabilization times (indicated below) with pepsin were tested for different samples as follows:
Methanol fixed only (20 minutes) (control)
4% PFA fixed on slide (30 minutes) (control)
4% PFA fixed on slide with decrosslinking (30 minutes)
2% PFA fixed on slide with decrosslinking (50 minutes)
1% PFA fixed on slide with decrosslinking (20 minutes)
4% PFA fixed cardiac perfused (20 minutes) (control)
4% PFA fixed cardiac perfused with decrosslinking (20 minutes)

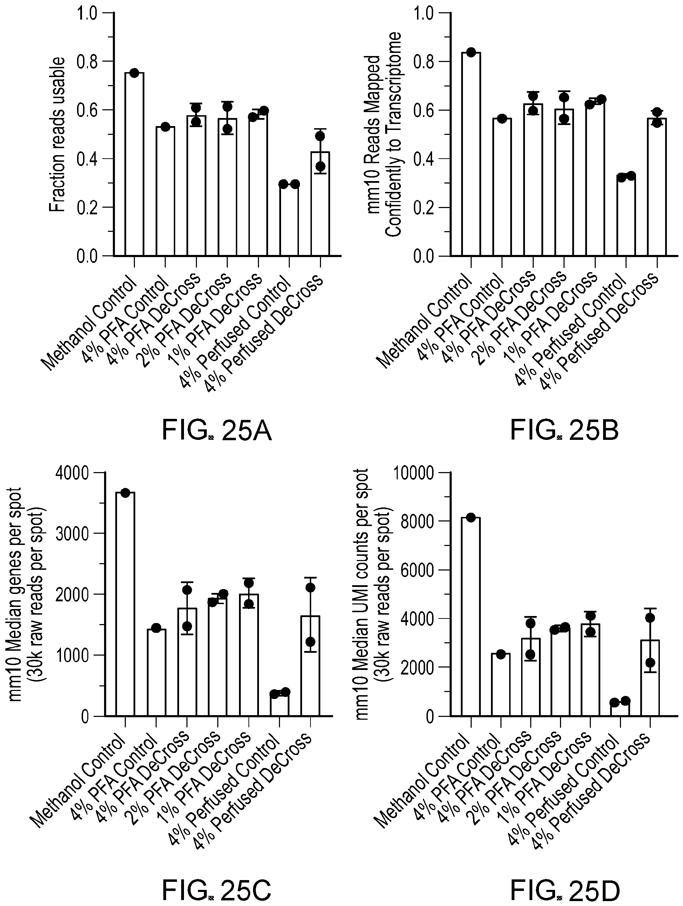

**FIGs. 25-D** show sequencing sensitivity metrics for the above listed samples. **FIG. 25A** shows the Fraction of usable read obtained from each sample. **FIG. 25B** shows the Reads Mapped Confidently to Transcriptome. **FIG. 25C** shows Median genes per spot (30k raw reads per spot). **FIG. 25D** shows Median UMI counts per spot (30k raw reads per spot). The data shown in FIGs. 25B-D was compared to the mouse mm 10 reference genome.

The data show the addition of a decrosslinking step improved the sensitivity in the 4% cardiac perfused PFA fixed samples relative to the 4% cardiac perfused PFA fixed control sample (no decrosslinking). Adding a decrosslinking step to the on slide PFA fixed mouse brain samples resulted in an increase in each of the sensitivity metrics relative to the 4% PFA control sample (no decrosslinking).

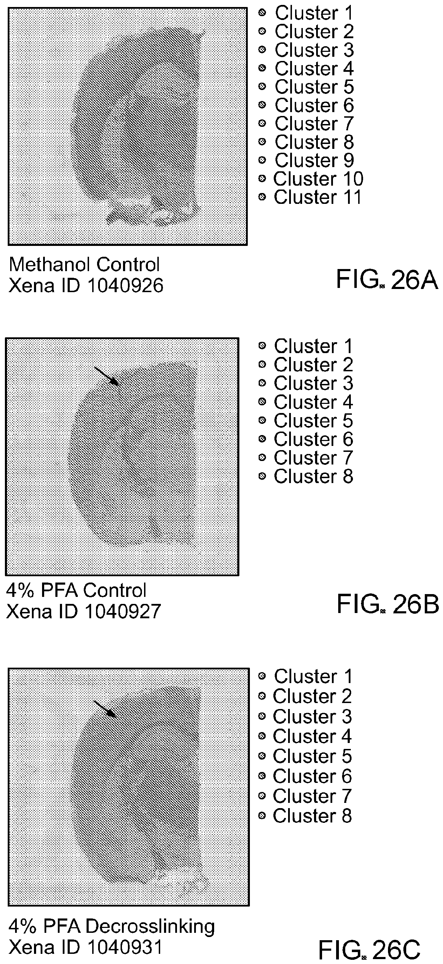

FIGs. 26A-C show spatial gene clustering performed using different mouse brain samples. The data shown in **FIG. 26A** is a methanol incubated sample only (control), the data shown in **FIG. 26B** is a 4% PFA on slide fixed mouse brain sample without decrosslinking, and the data shown in **FIG. 26C** is a 4% PFA on slide fixed mouse brain sample with decrosslinking as described above. **FIG. 26C** show improvements in spatial gene expression and spatial clustering, specifically the laminar distinction of the cortex (arrow).

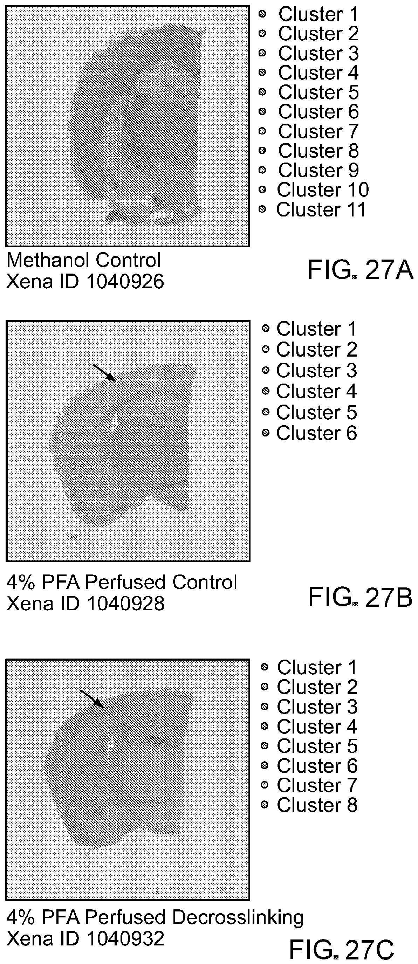

**FIGs. 27A-C** show spatial gene clustering in cardiac perfused samples. The data shown in **FIG. 27A** is a methanol incubated sample only (control), the data shown in **FIG. 27B** is a 4% PFA perfused control without decrosslinking, and the data shown in **FIG. 27C** is a 4% PFA perfused sample with decrosslinking as described above. The 4% perfused sample with decrosslinking shows increased sensitivity of spatial gene clustering (arrow) relative to the 5% PFA perfused control without decrosslinking.

### Example 12 - Random-based Second Strand Synthesis

Random primed second strand synthesis was tested to determine its effect on sensitivity in detecting analytes where a template switch oligonucleotide (TSO) was not incorporated during reverse transcription (e.g., first strand cDNA synthesis). Analytes (e.g., mRNA) were captured on a spatial array, reverse transcribed, and second strand synthesis was performed under different conditions. Reverse transcription and permeabilization were performed according to the Visium Spatial Gene Expression Reagent Kits User Guide (e.g., Rev C, dated June 2020). Multiple second strand synthesis conditions were tested including a sample where a TSO was included during reverse transcription, but with a primer including a TSO sequence followed by a random sequence (SEQ ID NO: 3) alone, a sample where a TSO was included during reverse transcription with a combination of a primer including a TSO sequence followed by a random sequence (SEQ ID NO: 3) and a TSO primer, and a third sample where no TSO was included during reverse transcription (control) followed with a primer including a TSO sequence followed by a random sequence (SEQ ID NO: 3).

Additionally, multiple DNA polymerases were tested with each of the second strand synthesis conditions described above. DNA polymerases Bst 2.0 and Bst 3.0 were each tested and both were found to be moderately thermostable with high strand displacement activity. Bst 3.0 showed higher reverse transcription activity and improved amplification and inhibitor tolerance. In all conditions, the primer(s) were kept at 4°C for 10 minutes to hybridize, followed by 60 minutes at 37°C for extension.

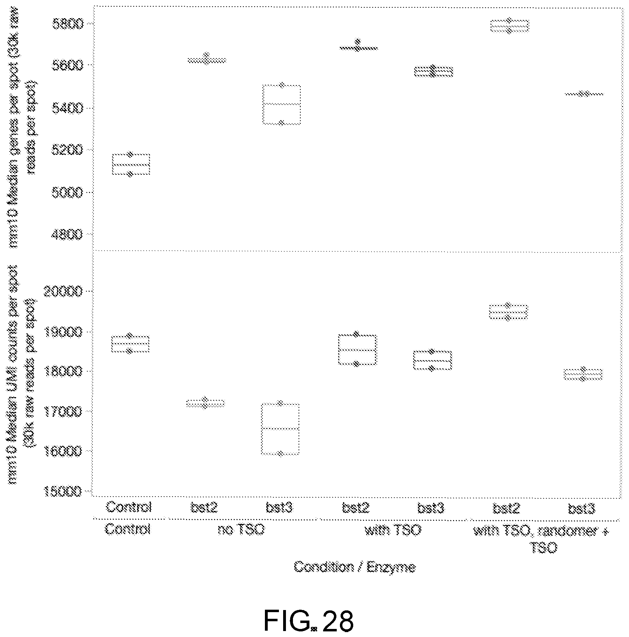

**FIG. 28** shows 30K raw reads metrics for various conditions shown on the x-axis. Bst 2.0 showed increased sensitivity compared to Bst 3.0 for second strand synthesis with a TSO sequence followed by a random sequence (SEQ ID NO: 3). **FIG. 28** also shows all second strand synthesis conditions with a TSO sequence followed by a random sequence ("randomer") showed increased median genes detected compared to control conditions. Approximately 5,800 genes were detected with Bst 2.0 and a combination of TSO and a TSO sequence followed by a random sequence (randomer) compared to 5150 genes for control, for an increase in sensitivity of around 13%. Median UMIs detected were similar across reverse transcription with TSO conditions.

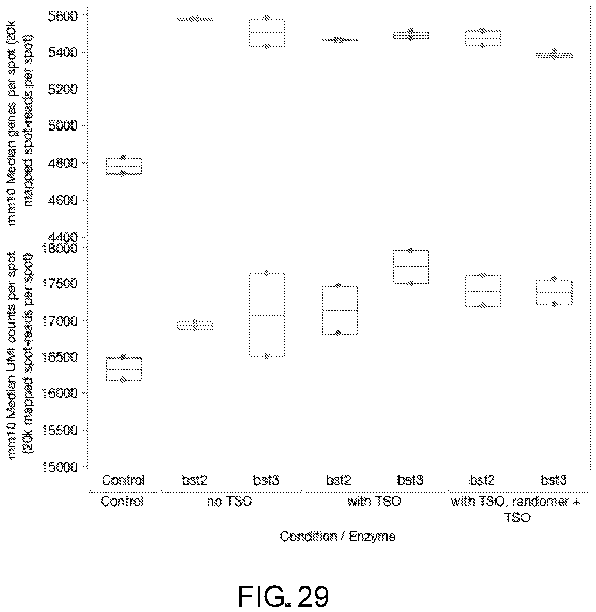

**FIG. 29** shows median genes and UMIs (mapped reads) for various conditions shown on the x-axis. When comparing mapped reads (20K), an increase in both median genes and UMIs was observed for second strand synthesis with a TSO sequence followed by a random sequence (randomer) and the combination of a TSO and a TSO sequence followed by a random sequence.

Collectively, the data demonstrate that a randomer-based second strand synthesis led to an increase in median genes (10-20%) detected, but a decrease in mapping and usable reads due more reads including the TSO sequence. The most successful condition tested was a combination of a TSO and a TSO including a random sequence (10 bp random sequence) with Bst 2.0. The data also shows that randomer-based second strand synthesis is compatible with TSO-free reverse transcription chemistry, but showed a slight reduction in sequencing quality and sensitivity compared to randomer-based second strand synthesis with TSO in the RT.

### Example 13 - Random-based Second Strand Synthesis

Random primed second strand synthesis was tested to determine whether sensitivity increased in detecting analytes where a template switch oligonucleotide (TSO) was not incorporated during reverse transcription (e.g., first strand cDNA synthesis). Analytes (e.g., mRNA) were captured on a spatial array, reverse transcribed, and second strand synthesis was performed under different conditions. Reverse transcription and permeabilization were performed according to the Visium Spatial Gene Expression Reagent Kits User Guide (e.g., Rev C, dated June 2020). Multiple second strand synthesis conditions were tested including a sample where a TSO was included during reverse transcription, but with a primer including a an 22 nt (x22) sequence followed by either a 6 nt random sequence (SEQ ID NO: 4) or a 10 nt random sequence (SEQ ID NO: 5), a sample where a TSO was included during reverse transcription with a combination of a primer including the x22 sequence followed by a either a 6 nt or 10 nt random sequence (SEQ ID NO: 4 and SEQ ID NO: 5, respectively) and a TSO primer, and a third sample where no TSO was included during reverse transcription (control) followed with a primer including the x22 sequence followed by either a 6 nt or 10 nt random sequence (SEQ ID NO: 4 and SEQ ID NO: 5, respectively). Reverse transcription was tested with two different cycling programs shown in Table 4 and Table 5 below. Second strand synthesis was performed with the reagents listed in Table 6 and tested with the x22 sequence with either a 6 nt random sequence (SEQ ID NO: 4) or a 10 nt random sequence (SEQ ID NO: 5).

**Table 4. Cycling Program at 37°C**

| **Step** | **Temp** | **Time** |
|---|---|---|
| Pre-chill | 4 °C | ∞ |
| Hybridization | 4 °C | 10 minutes |
| Extension | 37 °C | 60 minutes |
| Hold | 4 °C | ∞ |

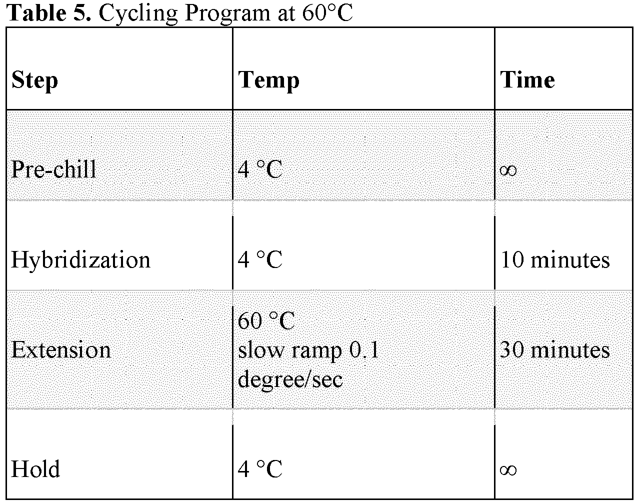

**Table 6. Second Strand Synthesis Reagents**

| **Reagent** | **Final Cone.** |
|---|---|
| Isothermal Buffer (NEB) | 1X |
| MgSO4 | 6 mM |
| dNTPs | 0.5 mM |
| Second Strand Primer | 5 µM |
| x22-randomer (6mer or 10mer) | 15 µM |
| Bst2.0 DNA Polymerase | 0.5 U/µL |

The data shown in Table 7 and 7A demonstrate that using an alternate sequence (x22 sequence) instead of the TSO sequence resulted in an increase (~10%) in both median genes and median UMIs detected compared to the combination of a TSO and a TSO sequence followed by a random sequence (Example 12). The data also demonstrate that the 37°C cycling program performed better than the 60°C slow modulation in terms of library quality and sensitivity. Minimal difference was detected between the 6 base pair and 10 base pair random sequences.

**Table 7.**

| Analysis ID | Condition | Temperature | Valid Barcodes | Reads Mapped Confidently to Transcriptome | Fraction reads usable |
|---|---|---|---|---|---|
| 1079344 | Control | Control | 96.9% | 86.5% | 79.8% |
| 1079345 | | | 96.9% | 84.9% | 78.7% |
| 1079352 | 6mer | 37°C | 96.6% | 84.2% | 78.3% |
| 1079353 | | | 96.5% | 84.4% | 77.0% |
| 1079354 | 10mer | | 96.7% | 85.2% | 78.3% |
| 1079355 | | | 96.7% | 85.6% | 76.9% |
| 1079356 | 6mer | 60°C | 96.5% | 81.9% | 75.7% |
| 1079357 | | | 96.4% | 81.9% | 74.6% |
| 1079358 | 10mer | | 96.6% | 83.5% | 75.9% |
| 1079359 | | | 96.7% | 83.3% | 77.3% |

**Table 7A.**

| Analysis ID | Condition | Temperature | Fraction reads with primer or homopolymer sequence | Fraction reads with any switch oligo sequence | Reads with low-support UMI | Fraction Reads in Spot |
|---|---|---|---|---|---|---|
| 1079344 | Control | Control | 4.1% | 7.2% | 0.5% | 95.6% |
| 1079345 | | | 5.9% | 10.9% | 0.5% | 95.9% |
| 1079352 | 6mer | 37°C | 4.8% | 6.8% | 1.0% | 96.9% |
| 1079353 | | | 4.4% | 6.8% | 0.9% | 95.2% |
| 1079354 | 10mer | | 4.3% | 6.0% | 0.8% | 95.6% |
| 1079355 | | | 4.2% | 6.7% | 0.8% | 93.5% |
| 1079356 | 6mer | 60°C | 4.7% | 4.5% | 0.4% | 95.9% |
| 1079357 | | | 4.9% | 5.4% | 0.4% | 94.5% |
| 1079358 | 10mer | | 5.7% | 6.3% | 0.6% | 94.3% |
| 1079359 | | | 5.5% | 5.3% | 0.6% | 96.2% |

## Claims

1. A method for de-crosslinking a fixed biological sample for determining the location of a target nucleic acid in the fixed biological sample, the method comprising:
(a) providing a spatial array comprising a plurality of capture probes, wherein a capture probe of the plurality of capture probes comprises: (i) a spatial barcode and (ii) a capture domain;
(b) de-crosslinking one or more crosslinks in the fixed biological sample, wherein the de-crosslinking comprises heating the fixed biological sample such that the capture domain hybridizes to a proxy of the target nucleic acid;
(c) contacting the fixed biological sample with:
(i) a first probe, wherein the first probe comprises in a 5' to 3' direction: a first functional domain and a first sequence that is substantially complementary to a portion of the target nucleic acid, and
(ii) a second probe, wherein the second probe comprises in a 5' to 3' direction: a 5' phosphate, a second sequence that is substantially complementary to a portion of the target nucleic acid and a capture sequence that is substantially complementary to the capture domain of the capture probe;
(d) hybridizing the first probe and the second probe to the target nucleic acid and ligating the 3' end of the first probe to the 5' end of the second probe to generate the proxy of the target nucleic acid;
(e) hybridizing the proxy of the target nucleic acid to the capture domain of the capture probe, extending the capture probe using the proxy as a template thereby generating an extended capture probe, and generating a second strand hybridized to the extended capture probe, wherein the second strand comprises a sequence complementary to the spatial barcode and the proxy of the target nucleic acid; and
(f) determining (i) the sequence of the spatial barcode, or a complement thereof, and (ii) all or a portion of the proxy of the target nucleic acid, or a complement thereof, and using the determined sequences of (i) and (ii) to identify the location of the target nucleic acid in the fixed biological sample.

2. The method of claim 1, wherein the fixed biological sample is a formalin-fixed paraffin-embedded (FFPE) biological sample or a paraformaldehyde fixed biological sample, optionally wherein the FFPE biological sample has undergone deparaffinization prior to step (b); or wherein the fixed biological sample is a formalin-fixed paraffin-embedded (FFPE) tissue section or a paraformaldehyde fixed tissue section, optionally wherein the FFPE tissue section has undergone deparaffinization prior to step (b).

3. The method of claim 1 or 2, wherein the de-crosslinking step comprises the use of Tris-EDTA (TE) buffer, wherein the TE buffer has a pH of about 7.5 to about 8.5 and wherein the TE buffer has a temperature of about 65°C to about 75°C, and is contacted with the FFPE biological sample for about 10 minutes to about 200 minutes.

4. The method of claim 1 or 2, wherein the de-crosslinking step comprises the use of Tris-HCl buffer with a pH of about 8.5 to 9.5 at a temperature of about 55°C to about 65°C, and is contacted with the paraformaldehyde fixed biological sample for about 10 minutes to about 200 minutes.

5. The method of any one of claims 1-4, further comprising, after the de-crosslinking in step (b), a step of permeabilizing the fixed biological sample, wherein the step of permeabilizing the fixed biological sample comprises the use of a protease, and optionally, wherein the protease is pepsin or proteinase K.

6. The method of any one of claims 1-5, wherein the plurality of capture probes comprises in a 5' to 3' direction: a spatial barcode, a unique molecular identifier, and a poly(T) capture domain.

7. The method of any one of claims 1-6, wherein the method further comprises, between steps (b) and (c), a step of incubating the fixed biological sample with a 5' to 3' single-stranded DNA exonuclease, and optionally, wherein between steps (b) and (c), a step of incubating the fixed biological sample with a 5' to 3' single-stranded DNA exonuclease and a double-stranded DNA endonuclease, wherein the single-stranded DNA exonuclease is T7 endonuclease or RecJ_{f}.

8. The method of any one of claims 1-7, wherein the method further comprises, incubating the fixed biological sample with a restriction endonuclease having an AT-rich restriction endonuclease recognition sequence.

9. The method of any one of claims 1-8, wherein the first functional domain is a first sequencing handle and wherein the capture sequence is substantially complementary to the capture domain, or a portion thereof.

10. The method of any one of claims 2-9, wherein the method further comprises, between steps (b) and (c), a step of treating the FFPE biological sample with an RNAse, and optionally, after step (d), removing unligated probes.

11. The method of any one of claims 1-10, wherein step (e) comprises: hybridizing a primer comprising at least a portion of the first functional domain to the capture probe and extending the primer to generate the second strand.

12. The method of any one of claims 1-11, wherein the method further comprises staining the fixed biological sample and imaging the fixed biological sample.

13. The method of any one of claims 1-12, wherein the determining in step (f) comprises sequencing.

14. The method of any one of claims 1-13, wherein the method further comprises blocking the capture domain prior to step (c).

15. The method of any one of claims 1-14, comprising after step (a) adding an analyte capture sequence to the target nucleic acid when the target nucleic acid sequence lacks an analyte capture sequence.

## Patentansprüche

1. Verfahren zum Entnetzen einer fixierten biologischen Probe zum Bestimmen des Ortes einer Ziel-Nukleinsäure in der fixierten biologischen Probe, wobei das Verfahren Folgendes umfasst:
(a) Bereitstellen eines räumlichen Arrays, umfassend eine Vielzahl von Einfangsonden, wobei eine Einfangsonde der Vielzahl von Einfangsonden umfasst: (i) einen räumlichen Barcode und (ii) eine Einfangdomäne;
(b) Entnetzen einer oder mehrerer Vernetzungen in der fixierten biologischen Probe, wobei das Entnetzen das Erwärmen der fixierten biologischen Probe umfasst, so dass die Einfangdomäne an einen Proxy der Ziel-Nukleinsäure hybridisiert;
(c) Inkontaktbringen der fixierten biologischen Probe mit:
(i) einer ersten Sonde, wobei die erste Sonde in 5'-zu-3'-Richtung umfasst: eine erste funktionelle Domäne und eine erste Sequenz, die im Wesentlichen komplementär zu einem Teil der Ziel-Nukleinsäure ist, und
(ii) einer zweiten Sonde, wobei die zweite Sonde in 5'-zu-3'-Richtung umfasst: ein 5'-Phosphat, eine zweite Sequenz, die im Wesentlichen komplementär zu einem Teil der Ziel-Nukleinsäure ist, und eine Einfangsequenz, die im Wesentlichen komplementär zu der Einfangdomäne der Einfangsonde ist;
(d) Hybridisieren der ersten Sonde und der zweiten Sonde an die Ziel-Nukleinsäure und Ligieren des 3'-Endes der ersten Sonde an das 5'-Ende der zweiten Sonde, um den Proxy der Ziel-Nukleinsäure zu erzeugen;
(e) Hybridisieren des Proxys der Ziel-Nukleinsäure an die Einfangdomäne der Einfangsonde, Verlängern der Einfangsonde unter Verwendung des Proxys als Matrize, wodurch eine verlängerte Einfangsonde erzeugt wird, und Erzeugen eines Zweitstrangs, der an die verlängerte Einfangsonde hybridisiert ist, wobei der Zweitstrang eine Sequenz umfasst, die zu dem räumlichen Barcode und dem Proxy der Ziel-Nukleinsäure komplementär ist; und
(f) Bestimmen (i) der Sequenz des räumlichen Barcodes oder eines Komplements davon und (ii) der Gesamtheit oder eines Teils des Proxys der Ziel-Nukleinsäure oder eines Komplements davon, und Verwenden der bestimmten Sequenzen von (i) und (ii), um den Ort der Ziel-Nukleinsäure in der fixierten biologischen Probe zu identifizieren.

2. Verfahren nach Anspruch 1, wobei die fixierte biologische Probe eine Formalin-fixierte, in Paraffin eingebettete (FFPE) biologische Probe oder eine Paraformaldehyd-fixierte biologische Probe ist, wobei optional die biologische FFPE-Probe vor Schritt (b) einer Entparaffinierung unterzogen wurde; oder wobei die fixierte biologische Probe ein Formalin-fixierter, in Paraffin eingebetteter (FFPE) Gewebeschnitt oder ein Paraformaldehyd-fixierter Gewebeschnitt ist, wobei optional der FFPE-Gewebeschnitt vor Schritt (b) einer Entparaffinierung unterzogen wurde.

3. Verfahren nach Anspruch 1 oder 2, wobei der Entnetzungsschritt die Verwendung von Tris-EDTA (TE)-Puffer umfasst, wobei der TE-Puffer einen pH-Wert von etwa 7,5 bis etwa 8,5 aufweist und wobei der TE-Puffer eine Temperatur von etwa 65 °C bis etwa 75 °C aufweist und für etwa 10 Minuten bis etwa 200 Minuten mit der biologischen FFPE-Probe in Kontakt gebracht wird.

4. Verfahren nach Anspruch 1 oder 2, wobei der Entnetzungsschritt die Verwendung von Tris-HCl-Puffer umfasst, der einen pH-Wert von etwa 8,5 bis 9,5 bei einer Temperatur von etwa 55 °C bis etwa 65 °C aufweist und für etwa 10 Minuten bis etwa 200 Minuten mit der Paraformaldehyd-fixierten biologischen Probe in Kontakt gebracht wird.

5. Verfahren nach einem der Ansprüche 1-4, ferner umfassend, nach der Entnetzung in Schritt (b), einen Schritt des Permeabilisierens der fixierten biologischen Probe, wobei der Schritt des Permeabilisierens der fixierten biologischen Probe die Verwendung einer Protease umfasst, und wobei die Protease optional Pepsin oder Proteinase K ist.

6. Verfahren nach einem der Ansprüche 1-5, wobei die Vielzahl von Einfangsonden in 5'-zu-3'-Richtung umfasst: einen räumlichen Barcode, einen eindeutigen molekularen Identifikator und eine Poly(T)-Einfangdomäne.

7. Verfahren nach einem der Ansprüche 1-6, wobei das Verfahren ferner, zwischen den Schritten (b) und (c), einen Schritt des Inkubierens der fixierten biologischen Probe mit einer 5'-zu-3'-Einzelstrang-DNA-Exonuklease umfasst, und optional wobei zwischen den Schritten (b) und (c) ein Schritt des Inkubierens der fixierten biologischen Probe mit einer 5'-zu-3'-Einzelstrang-DNA-Exonuklease und einer Doppelstrang-DNA-Endonuklease erfolgt, wobei die Einzelstrang-DNA-Exonuklease T7-Endonuklease oder RecJ_{f} ist.

8. Verfahren nach einem der Ansprüche 1-7, wobei das Verfahren ferner das Inkubieren der fixierten biologischen Probe mit einer Restriktionsendonuklease, aufweisend eine AT-reiche Restriktionsendonuklease-Erkennungssequenz, umfasst.

9. Verfahren nach einem der Ansprüche 1-8, wobei die erste funktionelle Domäne ein erster Sequenzierungs-Stiel ist und wobei die Einfangsequenz im Wesentlichen komplementär zu der Einfangdomäne oder einem Teil davon ist.

10. Verfahren nach einem der Ansprüche 2-9, wobei das Verfahren ferner, zwischen den Schritten (b) und (c), einen Schritt der Behandlung der biologischen FFPE-Probe mit einer RNAse und optional, nach Schritt (d), das Entfernen nicht-ligierter Sonden umfasst.

11. Verfahren nach einem der Ansprüche 1-10, wobei Schritt (e) umfasst: Hybridisieren eines Primers, der mindestens einen Teil der ersten funktionellen Domäne umfasst, an die Einfangsonde und Verlängern des Primers, um den Zweitstrang zu erzeugen.

12. Verfahren nach einem der Ansprüche 1-11, wobei das Verfahren ferner das Färben der fixierten biologischen Probe und das Abbilden der fixierten biologischen Probe umfasst.

13. Verfahren nach einem der Ansprüche 1-12, wobei das Bestimmen in Schritt (f) eine Sequenzierung umfasst.

14. Verfahren nach einem der Ansprüche 1-13, wobei das Verfahren ferner das Blockieren der Einfangdomäne vor dem Schritt (c) umfasst.

15. Verfahren nach einem der Ansprüche 1-14, das nach dem Schritt (a) das Hinzufügen einer Analyt-Einfangsequenz an die Ziel-Nukleinsäure umfasst, wenn der Ziel-Nukleinsäuresequenz eine Analyt-Einfangsequenz fehlt.

## Revendications

1. Procédé de dé-réticulation d'un échantillon biologique fixé pour déterminer l'emplacement d'un acide nucléique cible dans l'échantillon biologique fixé, le procédé comprenant :
(a) la fourniture d'un réseau spatial comprenant une pluralité de sondes de capture, dans lequel une sonde de capture de la pluralité de sondes de capture comprend : (i) un code-barres spatial et (ii) un domaine de capture ;
(b) la dé-réticulation d'une ou plusieurs réticulations dans l'échantillon biologique fixé, dans lequel la dé-réticulation comprend le chauffage de l'échantillon biologique fixé de sorte que le domaine de capture s'hybride à un proxy de l'acide nucléique cible ;
(c) la mise en contact de l'échantillon biologique fixé avec :
(i) une première sonde, dans lequel la première sonde comprend dans une direction 5' à 3' : un premier domaine fonctionnel et une première séquence qui est sensiblement complémentaire à une partie de l'acide nucléique cible, et
(ii) une deuxième sonde, dans lequel la deuxième sonde comprend dans une direction de 5' à 3' : un 5' phosphate, une deuxième séquence qui est sensiblement complémentaire à une partie de l'acide nucléique cible et une séquence de capture qui est sensiblement complémentaire au domaine de capture de la sonde de capture ;
(d) l'hybridation de la première sonde et de la deuxième sonde à l'acide nucléique cible et la ligature de l'extrémité 3' de la première sonde à l'extrémité 5' de la deuxième sonde pour générer le proxy de l'acide nucléique cible ;
(e) l'hybridation du proxy de l'acide nucléique cible au domaine de capture de la sonde de capture, l'extension de la sonde de capture en utilisant le proxy comme matrice, générant ainsi une sonde de capture étendue, et générant un deuxième brin hybridé à la sonde de capture étendue, dans lequel le deuxième brin comprend une séquence complémentaire au code-barres spatial et au proxy de l'acide nucléique cible ; et
(f) la détermination (i) de la séquence du code-barres spatial ou d'un complément de celui-ci, et (ii) de la totalité ou d'une partie du proxy de l'acide nucléique cible, ou d'un complément de celui-ci, et l'utilisation des séquences déterminées de (i) et (ii) pour identifier l'emplacement de l'acide nucléique cible dans l'échantillon biologique fixé.

2. Procédé selon la revendication 1, dans lequel l'échantillon biologique fixé est un échantillon biologique fixé au formol et incorporé dans de la paraffine (FFPE) ou un échantillon biologique fixé au paraformaldéhyde, éventuellement dans lequel l'échantillon biologique FFPE a subi un déparaffinage avant l'étape (b) ; ou dans lequel l'échantillon biologique fixé est une section de tissu fixée au formol et incorporée dans de la paraffine (FFPE) ou une section de tissu fixée au paraformaldéhyde, éventuellement dans lequel la section de tissu FFPE a subi un déparaffinage avant l'étape (b).

3. Procédé selon la revendication 1 ou 2, dans lequel l'étape de dé-réticulation comprend l'utilisation d'un tampon Tris-EDTA (TE), dans lequel le tampon TE a un pH d'environ 7,5 à environ 8,5 et dans lequel le tampon TE a une température d'environ 65 °C à environ 75 °C, et est mis en contact avec l'échantillon biologique FFPE pendant environ 10 minutes à environ 200 minutes.

4. Procédé selon la revendication 1 ou 2, dans lequel l'étape de dé-réticulation comprend l'utilisation d'un tampon Tris-HCl doté d'un pH d'environ 8,5 à 9,5 à une température d'environ 55 °C à environ 65 °C, et est mis en contact avec l'échantillon biologique fixé au paraformaldéhyde pendant environ 10 minutes à environ 200 minutes.

5. Procédé selon l'une quelconque des revendications 1-4, comprenant en outre, après la dé-réticulation à l'étape (b), une étape de perméabilisation de l'échantillon biologique fixé, dans lequel l'étape de perméabilisation de l'échantillon biologique fixé comprend l'utilisation d'une protéase et, éventuellement, dans lequel la protéase est une pepsine ou une protéinase K.

6. Procédé selon l'une quelconque des revendications 1-5, dans lequel la pluralité de sondes de capture comprend une direction de 5' à 3' : un code-barres spatial, un identifiant moléculaire unique et un domaine de capture poly(T).

7. Procédé selon l'une quelconque des revendications 1-6, dans lequel le procédé comprend en outre, entre les étapes (b) et (c), une étape d'incubation de l'échantillon biologique fixé avec un exonucléase d'ADN à simple brin de 5' à 3', et, éventuellement, dans lequel, entre les étapes (b) et (c), une étape d'incubation de l'échantillon biologique fixé avec une exonucléase d'ADN à simple brin de 5' à 3' et une endonucléase d'ADN à double brin, dans lequel l'exonucléase d'ADN à simple brin est une endonucléase T7 ou RecJ_{f}.

8. Procédé selon l'une quelconque des revendications 1-7, dans lequel le procédé comprend en outre l'incubation de l'échantillon biologique fixé avec une endonucléase de restriction ayant une séquence de reconnaissance de l'endonucléase de restriction AT-Rich.

9. Procédé selon l'une quelconque des revendications 1-8, dans lequel le premier domaine fonctionnel est une première poignée de séquençage et dans lequel la séquence de capture est sensiblement complémentaire au domaine de capture, ou à une partie de celui-ci.

10. Procédé selon l'une quelconque des revendications 2-9, dans lequel le procédé comprend en outre, entre les étapes (b) et (c), une étape de traitement de l'échantillon biologique FFPE avec une RNAse, et éventuellement, après l'étape (d), le retrait des sondes non ligaturées.

11. Procédé selon l'une quelconque des revendications 1-10, dans lequel l'étape (e) comprend : l'hybridation d'une amorce comprenant au moins une partie du premier domaine fonctionnel à la sonde de capture et l'extension de l'amorce pour générer le deuxième brin.

12. Procédé selon l'une quelconque des revendications 1-11, dans lequel le procédé comprend en outre la coloration de l'échantillon biologique fixé et l'imagerie de l'échantillon biologique fixé.

13. Procédé selon l'une quelconque des revendications 1-12, dans lequel la détermination à l'étape (f) comprend un séquençage.

14. Procédé selon l'une quelconque des revendications 1-13, dans lequel le procédé comprend en outre le blocage du domaine de capture avant l'étape (c).

15. Procédé selon l'une quelconque des revendications 1-14, comprenant après l'étape (a) l'ajout d'une séquence de capture d'analyte à l'acide nucléique cible lorsque la séquence d'acide nucléique cible est dépourvue d'une séquence de capture d'analyte.
